# FASCICULE DE BREVET EUROPEEN

(11) **EP 3 274 005 B1**
(45) Date de publication et mention de la délivrance du brevet: **04.12.2019**
(21) Numéro de dépôt: 16713834.6
(22) Date de dépôt: 25.03.2016
(51) Int. Cl.: A61L 27/24, A61L 27/60, A61L 27/38, A61F 2/10, C12N 5/00

(54) **PROCÉDÉ DE RECONSTRUCTION DE PEAU**
VERFAHREN ZUR REKONSTRUKTION VON HAUT
METHOD FOR SKIN RECONSTRUCTION

(30) Priorité: 26.03.2015 EP 15305441
(43) Date de publication de la demande: 31.01.2018
(73) Titulaire: Université de Bordeaux, 33000 Bordeaux (FR); Inserm, 75013 Paris (FR); Centre Hospitalier Universitaire de Bordeaux, 33404 Talence (FR)
(72) Inventeur: CASOLI, Vincent, 33950 Lège-Cap-Ferret (FR); CARIO-ANDRÉ, Muriel, 33600 Pessac (FR); LEPIVERT, Jean-Christophe, 33000 Bordeaux (FR)
(74) Mandataire: Novagraaf Technologies
(86) Numéro de dépôt international: PCT/EP2016/056700
(87) Numéro de publication internationale: WO 2016/151133

(56) Documents cités:
- WO-A2-96/19099
- US-A- 5 755 814
- US-A1- 2009 298 042
- NIIYAMA HAYATO ET AL: "Development of novel wound dressing composed of hyaluronic acid and collagen sponge containing epidermal growth factor and vitamin C derivative", JOURNAL OF ARTIFICIAL ORGANS, SPRINGER VERLAG, TOKYO, JP, vol. 17, no. 1, 1 décembre 2013 (2013-12-01), pages 81-87, XP035355413, ISSN: 1434-7229, DOI: 10.1007/S10047-013-0737-X [extrait le 2013-12-01]

## Description

### Domaine technique

La présente invention se rapporte à un procédé de préparation d'un substitut de peau, à un substitut de peau animale, de préférence de mammifère et/ou humaine, susceptible d'être obtenu par la mise en œuvre du procédé et à un kit pour la mise en œuvre du procédé.

La présente invention se rapporte également à un greffon constitué d'un substitut de peau et son utilisation comme moyen de traitement d'un désordre cutané et/ou d'une perte de substance cutanée.

La présente invention trouve une application notamment dans les domaines pharmacologique, médical, clinique.

Dans la description ci-dessous, les références entre crochets ([ ]) renvoient à la liste des références présentées à la fin du texte.

### État de la technique

La peau est un organe très complexe comprenant une structure stratifiée très particulière. Elle comprend trois parties principales :
- une partie superficielle, la plus mince, nommée épiderme,
- une partie interne plus épaisse, le derme, auquel est rattaché l'épiderme, et
- une couche plus profonde, l'hypoderme.

Elle assure notamment une barrière entre les milieux extérieurs et le milieu interne de nombreux mammifères, dont en particulier l'être humain. De par cette fonction de « barrière », la peau assure naturellement la protection de l'organisme tout en assurant une communication entre celui-ci et l'environnement extérieur. La peau constitue le premier organe de défense vis-à-vis de toute agression.

Les agressions subies par la peau sont multiples, il peut s'agir par exemple d'agressions liées aux U.V. susceptibles d'entraîner des réactions inflammatoires/altérations cellulaires à l'origine de cancers, des agressions physiques telles que des brûlures, des scarifications, par exemple dues à des objets contondants, des agressions chimiques, par exemple liées à des produits chimiques, par exemple des détergents. Ces différentes agressions peuvent induire notamment des désordres cutanés susceptibles de modifier la structure de la peau, d'altérer sa coloration et/ou de provoquer l'apparition de plaies cutanées.

Il est en effet connu que des produits chimiques et/ou des molécules telles que l'hydroquinone, lorsqu'ils sont utilisés à des doses importantes peuvent induire une forte dépigmentation, provoquer des cicatrices, des vergetures, des pathologies graves, par exemple du diabète, de l'hypertension, des cancers cutanés ou des complications systémiques, des désordres rénaux, un développement de la pilosité et provoquer de la barbe et une perturbation de l'odeur corporelle. Lorsque la décoloration va au-delà de l'effet désiré ces conséquences peuvent être irréversibles. La principale solution reste alors la greffe de peau afin d'essayer de « retrouver » un aspect normal.

D'autres éléments peuvent être à l'origine d'une altération de la peau, par exemple des paramètres d'ordre génétique et/ou liés à des troubles endocriniens et/ou auto-immuns systémiques. Il peut s'agir notamment de pathologies provoquant un désordre pigmentaire tel que le vitiligo, l'hypermélanose, l'hypomélanose ou un naevus. Un des procédés de traitement de tels désordres comprend la greffe de peau et/ou l'implantation de cellules pigmentaires. Toutefois, ces procédés présentent des efficacités relatives souvent liées à la stabilité des peaux et/ou cellules pigmentaires appliquées.

La peau peut également être blessée, par exemple par des éléments extérieurs tels que des objets contondants, provoquant des plaies plus ou moins profondes. La peau présente des capacités de régénération et de cicatrisation très importantes permettant, la plupart du temps, une cicatrisation à plus ou moins long terme. Le temps et/ou la capacité de cicatrisation pourront dépendre notamment de la profondeur/étendue de la plaie et également de l'état physiologique de l'individu. En effet, dans le cas de plaies profondes, celles-ci peuvent représenter des « portes ouvertes » à des pathogènes nécessitant une surveillance accrue et/ou la nécessité d'intervention afin de fermer la plaie, par exemple avec des points de suture ou en y appliquant une greffe de peau afin de permettre notamment à la peau de se régénérer, en particulier de réparer les différentes parties lésées.

Il existe dans l'état de la technique des substituts de derme artificiels permettant notamment de manière temporaire la formation d'un environnement favorable pour la régénération de la peau. La structure et l'environnement formés par ces substituts sont souvent proches de ceux du derme. Toutefois, ces substituts sont des dispositifs médicaux, essentiellement synthétiques et coûteux.

Il existe également dans l'état de la technique des systèmes de cultures de cellules, notamment de kératinocytes, obtenues à partir d'un échantillon de peau d'un individu formant après plusieurs semaines une culture qui peut être déposée/vaporisée sur une plaie, par exemple une plaie chronique ou une brûlure afin de favoriser sa cicatrisation.

Toutefois, ces procédés et substituts ne constituent pas en tant que tels des substituts de peau comprenant les cellules majoritaires constitutives de la peau, telles que les fibroblastes, les kératinocytes et les mélanocytes. En particulier, ces substituts ne comprennent pas de mélanocytes et ne permettent donc pas d'obtenir des substituts de peau pigmentés et/ou susceptibles d'être pigmentés. De plus, ces substituts ne peuvent pas être utilisés par exemple pour le traitement de désordres pigmentaires, le traitement des plaies profondes, par exemple dues à des interventions chirurgicales, des accidents provoquant par exemple une perte importante de peau pouvant aller jusqu'à la perte totale de peau.

Par ailleurs, les procédés d'obtention des substituts de derme disponibles sont des procédés « lents » qui ne permettent pas, par exemple, l'obtention de substituts dans des délais compatibles pour le traitement de plaies particulières telles que des brûlures profondes.

Enfin, la majorité des substituts de peau disponibles dans le commerce sont des produit fragiles tant au niveau structurel que épidémiologique. Aussi, ces produits sont souvent de petite taille afin, notamment d'éviter une éventuelle déchirure du produit lors de sa manipulation.

Il existe également dans l'état de la technique des systèmes/procédés de préparation de substituts de peau, par exemple tel que décrit dans le document US 5 755 814 comprenant notamment la culture de cellules, notamment de fibroblastes, et/ou d'un mélange de mélanocytes et kératinocytes. Toutefois, ces systèmes/procédés ne permettent pas l'obtention de substitut avec une structure identique à celle de la peau in-vivo. En outre, les substituts obtenus par ces procédés présentent une parakératose, anomalie de différentiation de la peau, correspondant à une maturation anormale de la kératine au niveau de la couche cornée ne permettant donc pas leurs utilisations dans le traitement de désordres cutanés et/ou de pertes de substance cutanée. Enfin, les systèmes/procédés de préparation de substituts de peau connus utilisent notamment des milieux comprenant des composés incompatibles avec une utilisation clinique, par exemple de l'extrait pituitaire de bovin.

Il existe donc un réel besoin de trouver un procédé de préparation d'un substitut de peau palliant ces défauts, inconvénients et obstacles de l'art antérieur, en particulier un procédé permettant d'obtenir un substitut de peau comprenant les cellules majoritaires constitutives de la peau, en particulier les fibroblastes, les kératinocytes et les mélanocytes, pour réduire les coûts et le temps de préparation du substitut.

Il existe également un réel besoin dans l'état de la technique de trouver un procédé permettant la production d'un modèle de peau reproductible et fiable.

Il existe en outre un réel besoin de trouver un nouveau substitut de peau utilisable pour le traitement de désordres cutanés et/ou de pertes de substance cutanée.

Il existe encore un réel besoin de trouver un nouveau substitut de peau facilement manipulable et ne présentant pas un fort risque de déchirure lors de sa manipulation.

### Description de l'invention

La présente invention a précisément pour but de répondre à ce besoin en fournissant un procédé de préparation d'un substitut de peau comprenant les étapes suivantes :
**a.** culture de fibroblastes dans un milieu M1 de culture de fibroblastes ;
**b.** ensemencement d'une matrice comprenant du collagène avec des fibroblastes issus de l'étape a ;
**c.** culture des fibroblastes ensemencés dans la matrice comprenant du collagène dans un milieu M2 de culture de fibroblastes comprenant de l'acide ascorbique ou un ascorbate ou un dérivé de ceux-ci, la matrice et les fibroblastes cultivés formant un substitut de derme ;
**d.** culture de mélanocytes dans un milieu M3 de culture de mélanocytes ;
**e.** culture de kératinocytes dans un milieu M4 de culture de kératinocytes ;
**f.** mélange de mélanocytes obtenus à l'étape d avec des kératinocytes obtenus à l'étape e ;
**g.** ensemencement du substitut de derme obtenu à l'étape c par le mélange obtenu à l'étape f ; dans lequel l'ensemencement à l'étape g est réalisé avec un ratio (kératinocytes+mélanocytes)/fibroblastes de 9 à 19, et
**h.** culture du substitut de derme ensemencé à l'étape g dans un milieu M5 de culture de peau formant ainsi le substitut de peau.

Dans la présente, le terme "comprendre" peut signifier tout aussi bien "inclure", "contenir" ou "englober" d'une part que "constituer de" ou "consister en" d'autre part.

Dans la présente le substitut de derme obtenu selon le procédé de l'invention est un tissu complet reproduisant les caractéristiques d'un derme *in vivo*, à savoir comprenant des macromolécules de type protéique, en particulier fibres de collagène, de glycosaminoglycanes, protéines et fibroblastes fonctionnels.

Dans la présente le substitut de peau obtenu selon le procédé de l'invention est un tissu complet reproduisant les caractéristiques d'une peau *in vivo*, à savoir comprenant un épithélium pluristratifié kératinisé comprenant des kératinocytes reproduisant un stratum basal, un stratum spinosum, un stratum granulosum et un stratum corneum histologiquement normaux, des mélanocytes basaux en contact avec un substitut de derme contenant des fibroblastes fonctionnels via une lame basale fonctionnelle.

Avantageusement, le procédé selon l'invention permet d'obtenir un substitut de peau comprenant une lame basale constituée notamment d'un mélange protéique sécrété par les cellules du substitut formant ainsi une jonction dermo-épidermique reproduisant les caractéristiques d'une peau *in vivo.*

Dans la présente, les fibroblastes, mélanocytes, kératinocytes susceptibles d'être utilisés dans le procédé de l'invention peuvent être tous fibroblastes, mélanocytes, kératinocytes connus de l'homme du métier. Il peut s'agir par exemple de fibroblastes, mélanocytes et/ou de kératinocytes obtenus à partir de banques de cellules, par exemple provenant de la Collection Nationale de Culture de Microorganisme (CNCM) de l'Institut Pasteur, 25 rue du Docteur Roux, F-75724 PARIS Cedex 15. Il peut s'agir également de fibroblastes, mélanocytes et/ou kératinocytes disponibles dans le commerce, par exemple les cellules commercialisées par la société Thermofischer scientific, la société CellnTec ou la société Promocell. Il peut s'agir également de fibroblastes, mélanocytes et/ou kératinocytes isolés à partir d'un échantillon biologique d'un animal, de préférence un mammifère et/ou d'un être humain, préalablement isolé. Les fibroblastes, mélanocytes et/ou kératinocytes peuvent être des fibroblastes, mélanocytes, kératinocytes isolés indépendamment d'une biopsie ou plusieurs biopsies. Les fibroblastes, mélanocytes et/ou kératinocytes peuvent être isolés indépendamment d'une biopsie ou plusieurs biopsies d'un individu, de préférence un mammifère et/ou un être humain, en vue d'une greffe dudit substitut de peau sur ledit patient. Il peut s'agir indépendamment de fibroblastes, mélanocytes et/ou kératinocytes autologues ou hétérologues d'un individu.

Dans la présente, avantageusement au moins deux types cellulaires parmi les trois types cellulaires représentés par les fibroblastes, mélanocytes et kératinocytes sont autologues d'un individu.

Dans un mode de réalisation particulier, les fibroblastes, mélanocytes et kératinocytes sont avantageusement des cellules autologues d'un individu.

Les fibroblastes, mélanocytes et/ou kératinocytes peuvent être isolés indépendamment d'une biopsie ou plusieurs biopsies provenant, par exemple d'une peau caucasienne, asiatique ou africaine, de différents sites anatomiques, par exemple du dos, visage, sein, dos des mains, paumes d'un être humain

Il peut s'agir indépendamment de fibroblastes, mélanocytes et/ou de kératinocytes isolés indépendamment de biopsies de peau, de préférence d'un être humain, présentant une ou plusieurs pathologies cutanées, par exemple des taches de vieillesse (lentigo actinique), melasma, vitiligo, naevus ou mélanome.

Il peut s'agir également de fibroblastes, mélanocytes et/ou kératinocytes indépendamment génétiquement modifiés, par exemple avec des rétrovirus, lentivirus, des adénovirus, des adeno associated virus (AAV). Il peut s'agir par exemple de fibroblastes, mélanocytes et/ou kératinocytes surexprimant indépendamment au moins une protéine, par exemple une protéine choisie parmi le collagène VII, les kératines 5, 14, catalase, SIRT6 et/ou sous exprimant au moins une protéine, par exemple via la technique des petits ARN en épingle à cheveux (shRNA) ou des petits ARN interférant (siRNA), par exemple le collagène VII, HIF1, CCN3. Il peut s'agir par exemple de fibroblastes, mélanocytes et/ou kératinocytes indépendamment génétiquement modifiés tel que décrit dans Pendaries V et al, JID 2012 [1]; Petek LM et al Mol ther 2010 [2]. Il peut s'agir par exemple de fibroblastes, mélanocytes et/ou kératinocytes indépendamment génétiquement modifiés ou non, par exemple la cellule Ker-CT identifiée sous la référence ATCC CRL-4048, la cellule TelCOFS02MA identifié sous la référence ATCC CRL-4005.

Il peut s'agir également de fibroblastes, mélanocytes et/ou kératinocytes issus de lignée cellulaire, par exemple la lignée kératinocytaire HaCaT, la lignée fibroblastique WS1.

De façon préférée, les fibroblastes susceptibles d'être utilisés ne comprennent pas de fibroblastes irradiés 3T3.

Il peut s'agir également de fibroblastes, mélanocytes et/ou kératinocytes indépendamment obtenus à partir de cellules souches adultes, de cellules souches pluripotentes induites, par exemple par maintien desdites cellules souches adultes et/ou de cellules souches pluripotentes induites via par exemple l'introduction de gènes Oct3/4, Sox 2, KLF4, c-Myc puis différenciées grâce à des cocktails de facteurs, par exemple l'acide rétinoïque et/ou le BMP-4, en une lignée cellulaire. Il peut s'agir également de cellules souches adultes et/ou de cellules souches pluripotentes induites par des techniques non virales basées sur l'utilisation de nanoparticules, par exemple l'arginine-terminated polyamidoamine nanoparticules. L'homme du métier de par ses connaissances générales saura choisir le procédé et/ou les cellules. Il peut s'agir par exemple de fibroblastes, mélanocytes et/ou kératinocytes obtenus par le procédé décrit dans Kogut et al Methods Mol Biol 2014 [3], dans Ohta et al, Methods Mol Biol, 2013 [4], et/ou dans Revilla et al, J Tissue Eng Regen Med, 2015 [5].

Dans la présente par milieu M1 de culture de fibroblastes on entend tout milieu connu de l'homme du métier adapté à la culture de fibroblastes. Il peut s'agir par exemple d'un milieu disponible dans le commerce, par exemple un milieu Dulbecco modified Eagle's minimal essential medium (DMEM) commercialisé par la société Gibco comprenant notamment un mélange d'acides aminés, de vitamines, de sels inorganiques, de sucres, par exemple du glucose, ou un milieu Fibrolife commercialisé par la société Cell Systems

**Tableau 1 : composition du milieu Dulbecco modified Eagle's minimal essential medium (DMEM)**

| **Composition** | **Concentration (mg/L)** |
|---|---|
| **Acides aminés** | |
| Glycine | 84 |
| L-Arginine hydrochlorure | 84 |
| L-Cystine 2HCl | 63 |
| L-Glutamine | 580 |
| L-Histidine hydrochlorure-H₂O | 42 |
| L-Isoleucine | 105 |
| L-Leucine | 105 |
| L-Lysine hydrochlorure | 146 |
| L-Méthionine | 30 |
| L-Phénylalanine | 66 |
| L-Serine | 42 |
| L-Thréonine | 95 |
| L-Tryptophane | 16 |
| L-Tyrosine | 72 |
| L-Valine | 94 |

| **Vitamines** | |
|---|---|
| Choline chlorure | 4 |
| D-Calcium pantothenate | 4 |
| Acide Folique | 4 |
| Niacinamide | 4 |
| Pyridoxine hydrochlorure | 4 |
| Riboflavine | 0,4 |
| Hydrochlorure de Thiamine | 4 |
| i-Inositol | 7,2 |

| **Sels inorganiques** | |
|---|---|
| Chlorure de Calcium (CaCl₂-2H₂O) | 264 |
| Ferric Nitrate (Fe(NO₃)₃"9H₂O) | 0,1 |
| Sulfate de Magnésium (MgSO₄-7H₂O | 200 |
| Chlorure de Potassium (KCl) | 400 |
| Bicarbonate de Sodium (NaHCO₃) | 3700 |
| Chlorure de Sodium (NaCl) | 6400 |
| Sodium Phosphate monobasic (NaH2PO4-2h2O | 141 |

| **Autres composés** | |
|---|---|
| D-Glucose (Dextrose) | 1000 |
| Sodium Pyruvate | 110 |

Dans la présente, le milieu M1 peut comprendre en outre des compléments, notamment du sérum de veau fœtal (SVF).

Dans la présente, le milieu M1 peut comprendre par exemple de 5 à 15% en poids, de 7,5 à 12,5% en poids, 10% en poids de sérum de veau fœtal (SVF) par rapport au poids total du milieu.

Dans la présente, le milieu M1 peut comprendre au moins un composé antifongique et/ou antibiotique. Il peut s'agir par exemple de tout composé antifongique et/ou antibiotique connu de l'homme du métier et/ou disponible dans le commerce. Il peut s'agir par exemple d'au moins un composé antifongique choisi dans le groupe comprenant l'Amphotéricine B, le kétoconazole et un mélange de ceux-ci. Il peut s'agir par exemple d'au moins un composé antibiotique choisi dans le groupe comprenant la pénicilline, la streptomycine, la ciprofloxacine et un mélange de ceux-ci.

Dans la présente, le milieu M1 peut comprendre de 0,1 à 10% en poids, de 0,5 à 5 % en poids, 1 % en poids d'antifongique par rapport au poids total du milieu.

Dans la présente, le milieu M1 peut comprendre de 0,1 à 10% en poids, de 0,5 à 5 % en poids, 1 % en poids d'antibiotiques par rapport au poids total du milieu.

Dans la présente le milieu M1 et/ou l'ensemble de ses constituants peu(ven)t être de grade clinique.

Par « de grade clinique » on désigne dans la présente le fait que le composant ou le milieu ait été reconnu par l'autorité compétente comme étant adapté pour une utilisation en clinique sur un territoire donné. Avantageusement dans la présente, lorsque le milieu est de grade clinique il ne comprend pas d'extrait pituitaire de bovin.

Dans la présente, l'étape a. de culture de fibroblastes peut être réalisée à une température comprise de 30 à 40°C, de 35 à 39°C, égale à 37°C.

Dans la présente, la durée de culture de fibroblastes de l'étape a. peut être comprise de 5 à 21 jours, de 5 à 15 jours, de 8 à 15 jours.

Dans la présente, la durée de culture de fibroblastes de l'étape a. peut être réalisée sous une atmosphère contrôlée comprenant de 5 à 10 % de CO₂, par exemple sous une atmosphère comprenant au moins 5% de CO₂.

Selon l'invention, l'étape a. de culture de fibroblastes peut être réalisée dans une étuve à une température de 30 à 40°C, de 32 à 40°C, égale à 37°C et sous une atmosphère contrôlée comprenant au moins 5% de CO₂

Selon l'invention, l'étape a. de culture de fibroblastes peut être réalisée dans tout contenant de culture adapté connu de l'homme du métier. Il peut s'agir d'une boite de pétri, d'un flacon de culture d'une contenance de 25 à 75 cm², de 25, 75 ou 175 cm².

Selon l'invention, les fibroblastes obtenus par culture selon l'étape a. peuvent former un tapis de cellules à confluence dans le contenant de culture. Par exemple, les fibroblastes peuvent être de 70 à 100% de confluence, de préférence à 100% de confluence.

Selon l'invention, lorsque la culture selon l'étape a. correspond à un tapis de cellules à confluence ou non, le procédé peut comprendre en outre :
- une étape a' d'élimination du milieu de culture, rinçage des cellules par une solution, élimination de la solution de rinçage,
- une étape a" de détachement des cellules par trypsinisation, et
- une étape a'" de culottage ou centrifugation.

Selon l'invention, dans l'étape a' l'élimination du milieu de culture peut être réalisée par tout procédé connu de l'homme du métier adapté. Il peut s'agir par exemple d'une aspiration du milieu, d'un retournement du contenant afin d'éliminer le milieu de culture.

Selon l'invention, dans l'étape a' le rinçage des cellules peut être réalisé par tout procédé connu de l'homme du métier, par exemple, par trempage, aspersion, incubation des cellules dans une solution de rinçage.

Dans la présente, par solution de rinçage on entend toute solution de rinçage de cellules connue de l'homme du métier. Il peut s'agir par exemple d'une solution tampon HBSS (Hank's Balanced Salt Solution) à pH compris de 7,2 à 7,4.

**Tableau 2 : composition du milieu HBSS**

| Composés | Poids moléculaire g/mol | Concentration (mg/L) | mM |
|---|---|---|---|
| **Sels inorganiques** | | | |
| Chlorure de potassium | 75 | 400 | 5,333335 |
| Phosphate de potassium monobasique (KH₂PO₄) | 136 | 60 | 0,4411 |
| Bicarbonate de Sodium | 84 | 350 | 4,16 |
| Chlorure de sodium | 58 | 8000 | 137,93 |
| Phosphate de Sodium dibasique anhydre (Na₂HP0₄) | 142 | 48 | 0,338 |

| **Autres composés** | | | |
|---|---|---|---|
| D glucose (Dextrose) | 180 | 1000 | 5,55 |
| Rouge phénolique | 376,4 | 10 | 0,0265 |

Il peut s'agir également d'une solution tampon disponible dans le commerce, par exemple un tampon phosphate salin (PBS), une solution équilibrée de Hank's commercialisée respectivement par la société Gibco, Sigma Aldrich, Lonza.

Selon l'invention, dans l'étape a' l'élimination de la solution de rinçage peut être réalisée par tout procédé connu de l'homme du métier adapté. Il peut s'agir par exemple d'une aspiration de la solution de rinçage, d'un retournement du contenant afin d'éliminer la solution de rinçage.

Selon l'invention, l'étape a" de trypsinisation peut être réalisée par immersion des cellules dans une solution tampon (ST) comprenant de la trypsine, suivie de l'ajout de Sérum de Veau Fœtal (SVF) afin d'arrêter la réaction enzymatique.

Selon l'invention, la solution tampon (ST) peut être toute solution tampon connue de l'homme du métier susceptible d'être utilisée dans un procédé de trypsinisation. Il peut s'agir par exemple d'un tampon phosphate salin (PBS), d'une solution équilibrée de Hank's commercialisée respectivement par la société Gibco, Sigma Aldrich, Lonza.

Selon l'invention la quantité de trypsine ajoutée dans la solution tampon (ST) peut être comprise entre 0,01 et 0,05 % en poids par rapport au poids total.

Selon l'invention la durée d'incubation dans la solution tampon comprenant de la trypsine avant ajout du SVF dans la solution tampon (ST) peut être comprise entre 2 et 10 min.

Selon l'invention la quantité de SVF ajoutée dans la solution tampon (ST) peut être comprise de 5 à 20 % en volume par rapport au volume total.

Selon l'invention, l'étape a'" de culottage peut être réalisée par tout procédé connu de l'homme du métier. Il peut s'agir par exemple d'une sédimentation ou une centrifugation à une vitesse de 800 à 1400 tours par minutes, par exemple égale à 1200 tours par minute.

Selon l'invention, l'étape a'" de centrifugation peut être réalisée pendant une durée de 4 à 10 min par exemple égale à 5 minutes.

Selon l'invention, l'étape a'" de culottage peut être réalisée par tout dispositif connu de l'homme du métier. Il peut s'agir par exemple d'une centrifugeuse rotative commercialisée par les sociétés Eppendorf ou Jouan.

Dans la présente par « matrice comprenant du collagène » on entend toute matrice comprenant du collagène connue de l'homme du métier susceptible d'être ensemencée par des cellules. Il peut s'agir par exemple d'une matrice de de collagène correspondant à un gel de collagène de type I non tendu, de préférence n'imposant pas d'organisation préférentielle des fibroblastes tel que décrit dans Bell et al, 1979 [6]. Il peut s'agir par exemple d'une matrice avec une densité / concentration de collagène, de préférence du collagène de type I, avec une surface de 25 à 500 cm². Il peut s'agir, par exemple d'une matrice comprenant du collagène disponible dans le commerce, par exemple il peut s'agir d'une matrice comprenant du collagène commercialisé par la société Integra.

Avantageusement, la matrice comprenant du collagène peut être une matrice de régénération dermique. La matrice de régénération dermique peut être notamment choisie parmi les matrices commercialisées sous les dénominations Integra (marque déposée) et Matriderm (marque déposée) par les sociétés Intégra Life Science Corporation et MedSkin Solutions Dr. Suwelack AG respectivement. Avantageusement, et contrairement aux autres matrices comprenant du collagène, les matrices de régénération dermique telles que celles citées ci-avant sont déjà modelées, ce qui favorise la reconstruction de l'équivalent de peau.

Dans un mode de réalisation, la matrice comprenant du collagène peut être une matrice comprenant du collagène réticulé et au moins un glycosaminoglycane, par exemple du 6-sulfate de chondroïtine. Il peut s'agir par exemple de la matrice Integra (marque déposée) commercialisée par la société Integralife Sciences et/ou de la matrice obtenue selon le procédé décrit dans le document Boyce ST et al 1988 [7].

Dans un autre mode de réalisation, la matrice comprenant du collagène peut être une matrice comprenant des fibres de collagène de structure native et de l'élastine. Par fibres de collagène de structure native on entend notamment des fibres non chimiquement réticulées. Il peut s'agir par exemple de la matrice Matriderm commercialisée par la société MedSkin Solutions Dr. Suwelack AG et/ou de la matrice obtenue selon le procédé décrit dans le document Hafemann et al, Burns 1999 [8].

Dans la présente, l'épaisseur de la matrice comprenant du collagène peut être de 1,0 à 3,0 mm (bornes incluses) avant ensemencement par les fibroblastes. Dans un mode de réalisation particulier, l'épaisseur de la matrice comprenant du collagène peut être strictement supérieure à 1,0 mm avant ensemencement par les fibroblastes.

Dans la présente, l'ensemencement de l'étape b peut être réalisé par tout procédé connu de l'homme du métier. Il peut s'agir par exemple d'une application, par exemple par aspersion d'un milieu de culture comprenant les fibroblastes sur la matrice, par dépôt par repiquage des cellules sur la matrice, par versement d'un milieu de culture comprenant les cellules en suspension, par impression 3D par exemple tel que décrit dans Wonhye Lee et al. « Multi-layered culture of human skin fibroblasts and keratinocytes through three-dimensional freeform fabrication." Biomaterials, 2009, March; 30(8):1587-95 [7].

Dans la présente, lorsque l'étape a comprend l'étape a", le procédé de l'invention peut comprendre avant l'étape b d'ensemencement une étape b1 de resuspension des cellules centrifugées dans le milieu M1.

Dans la présente, l'ensemencement de l'étape b d'une matrice comprenant du collagène peut être réalisé à une densité de 20 000 à 50 000 fibroblastes/cm², de préférence de 30 000 fibroblastes/cm² de surface de la matrice comprenant du collagène. Dans un mode de réalisation particulier, la densité de fibroblastes peut être strictement inférieure à 50 000 fibroblastes / cm² de matrice comprenant du collagène.

Dans la présente le milieu M2 de culture de fibroblastes peut être tout milieu connu de l'homme du métier adapté à la culture de fibroblastes. Il peut s'agir par exemple d'un milieu disponible dans le commerce, par exemple un milieu Dulbecco Modified Eagle's minimal essential medium (DMEM) comprenant notamment un mélange d'acides aminés, de vitamines, de sels inorganiques de sucres, par exemple du glucose.

Dans la présente, le milieu M1 peut comprendre en outre des compléments, notamment du sérum de veau fœtal (SVF).

Dans la présente, le milieu M2 peut comprendre de 5 à 15% en poids, de 7,5 à 12,5% en poids, 10% en poids de sérum de veau fœtal (SVF) par rapport au poids total du milieu.

Dans la présente, le milieu M2 peut comprendre au moins un composé antifongique et/ou antibiotique. Il peut s'agir par exemple de tout composé antifongique et/ou antibiotique connu de l'homme du métier et/ou disponible dans le commerce. Il peut s'agir par exemple d'au moins un composé antifongique choisi dans le groupe comprenant l'Amphotéricine B, du ketoconazole et d'un mélange de ceux-ci. Il peut s'agir par exemple d'au moins un composé antibiotique choisi dans le groupe comprenant la pénicilline, la streptomycine, la ciprofloxacine et un mélange de ceux-ci.

Dans la présente, le milieu M2 peut comprendre de 0,1 à 10% en poids, de 0,5 à 5 % en poids 1 % en poids d'antifongique par rapport au poids total du milieu.

Dans la présente, le milieu M2 peut comprendre de 0,1 à 10 % en poids, de 0,5 à 5 % en poids, égale à 1 % en poids d'antibiotiques par rapport au poids total du milieu.

Dans la présente, le milieu M2 peut comprendre en outre de l'acide ascorbique ou ascorbate ou un dérivé de ceux-ci. Par exemple, le milieu M2 peut comprendre de de l'acide ascorbique ou ascorbate à une concentration de 20 à 60 mg.mL⁻¹, par exemple de 30 à 55 mg.mL⁻¹, égale à 50 mg.mL⁻¹.

Dans la présente, le terme « dérivé » désigne tout dérivé de l'acide carboxylique ou du carboxylate connu par l'homme du métier. Par exemple, ce terme peut inclure des dérivés tels que des esters ou des anhydrides des acides correspondants.

Avantageusement, l'acide ascorbique permet notamment de favoriser le remodelage de la matrice comprenant du collagène en stimulant la synthèse de collagène par les fibroblastes.

Dans la présente le milieu M2 et/ou l'ensemble de ses constituants peu(ven)t être de grade clinique.

Dans la présente, l'étape c. de culture de fibroblastes peut être réalisée à une température comprise de 30 à 40°C, de 35 à 39°C, égale à 37°C.

Dans la présente, la durée de culture de l'étape c. de fibroblastes peut être de 5 à 12 jours, de 7 à 10 jours.

Dans la présente, la culture de l'étape c. de fibroblastes peut être réalisée sous une atmosphère contrôlée comprenant au moins 5% de CO₂.

Dans la présente, l'étape c. de culture des fibroblastes ensemencés dans la matrice comprenant du collagène peut comprendre :
- une première étape c' de culture de 18 à 28 heures en présence d'un milieu M2¹ de culture de fibroblastes ne comprenant ni acide ascorbique ni ascorbate, et
- une deuxième étape c" de culture, d'au moins 2 jours en présence d'un milieu M2² de culture de fibroblastes comprenant de l'acide ascorbique ou un ascorbate ou un dérivé de ceux-ci.

Dans ce mode de réalisation, le milieu M2¹ de culture de fibroblastes correspond au milieu M2 tel que défini ci-dessus ne comprenant ni acide ascorbique ni ascorbate ni dérivé de ceux-ci. Dans la présente le milieu M2¹ et/ou l'ensemble de ses constituants peu(ven)t être de grade clinique.

Dans ce mode de réalisation, le milieu M2² de culture de fibroblastes correspond au milieu M2 tel que défini ci-dessus comprenant de l'acide ascorbique ou un ascorbate ou un dérivé de ceux-ci. Dans la présente le milieu M2² et/ou l'ensemble de ses constituants peu(ven)t être de grade clinique.

Dans la présente, l'étape c'. de culture peut être réalisée à une température comprise de 30 à 45°C, de 35 à 39°C, égale à 37°C.

Dans la présente, la durée de culture de l'étape c'. peut être de 19 à 27 heures, par exemple de 24 heures.

Dans la présente, l'étape c". de culture peut être réalisée à une température comprise de 30 à 40°C, de 35 à 39°C, égale à 37°C.

Dans la présente, la durée de culture de l'étape c". peut être comprise entre 5 à 12 jours, égale à 7 jours.

Les inventeurs ont également démontré avantageusement que la matrice et les fibroblastes cultivés obtenus à l'étape c. forment une structure correspondant à un substitut de derme.

Avantageusement, les inventeurs ont également démontré que l'étape c' de culture correspond à une étape d'adhésion et de colonisation de la matrice par les fibroblastes et l'étape c" permet avantageusement un remodelage de la matrice comprenant les fibroblastes afin de former un substitut de derme. En particulier, la succession des étapes c' et c" avec l'utilisation respectivement des milieux M2¹ et M2² permettra avantageusement de former un substitut de derme dans lequel les fibroblastes ne prolifèrent pas mais colonisent la matrice comprenant du collagène tout en permettant avantageusement la production de collagène par les fibroblastes eux-mêmes permettant ainsi un remodelage du derme.

En d'autres termes le produit obtenu à l'issu de l'étape c. peut être avantageusement utilisé comme substitut de derme. En particulier, ce produit comprend toutes les caractéristiques physicochimiques du derme à partir duquel peuvent être issus les fibroblastes.

Selon l'invention, l'étape d. de culture de mélanocytes peut être réalisée dans tout contenant de culture adapté connu de l'homme du métier. Il peut s'agir d'une boite de pétri, d'un flacon de culture d'une contenance de 25 à 75 cm², de 25, 75 ou 175 cm².

Dans la présente le milieu M3 de culture de mélanocytes peut être tout milieu connu de l'homme du métier adapté à la culture de mélanocytes. Il peut s'agir par exemple d'un milieu disponible dans le commerce, par exemple un milieu disponible dans le commerce commercialisés par la société Promocell sous la référence « Melanocyte Medium M2 », « MBM » commercialisés par la société Promocell, dans un milieu MCDB 153 commercialisé par la société Sigma-Aldrich comprenant notamment un mélange d'acides aminés, de vitamines, de sels inorganiques de sucres, par exemple du glucose, tel que représenté dans le tableau 3 ci-dessous :

**Tableau 3 : composition du milieu MCDB 153**

| **Composition** | **Concentration en g.L⁻¹** |
|---|---|
| Ammonium Metavanadate | 0,000000585 |
| Chlorure de Calcium •Anhydre | 0,00333 |
| Cupric Sulfate•5 H₂O | 0,00000275 |
| Sulfate Ferreux•7 H₂O | 0,00139 |
| Chlorure de Magnésium | 0,05713 |
| Manganèse Sulfate | 0,000000151 |
| Molybdic Acide•4 H₂O (ammonium) | 0,00000124 |
| Nickel Chloride•6 H₂O | 0,00000012 |
| Potassium Chloride | 0,11183 |
| Sodium Acétate (anhydre) | 0,30153 |
| Chlorure de Sodium | 7,599 |
| Sodium Metasilicate•9 H₂O | 0,000142 |
| Sodium Phosphate Dibasique (anhydre) | 0,284088 |
| Sodium Sélénite | 0,0000038 |
| Stannous Chloride•2 H₂O | 0,000000113 |
| Zinc Sulfate•7 H₂O | 0,000144 |
| L-Alanine | 0,00891 |
| L-Arginine•HCl | 0,2107 |
| L-Asparagine• H₂O | 0,015 |
| L-Aspartique Acide | 0,00399 |
| L-Cystéine•HCl• H₂O | 0,04204 |
| L-Glutamique Acide | 0,01471 |
| L-Glutamine | 0,8772 |
| Glycine | 0,00751 |
| L-Histidine•HCl• H₂O | 0,01677 |
| L-Isoleucine | 0,001968 |
| L-Leucine | 0,0656 |
| L-Lysine•HCl | 0,01827 |
| L-Méthionine | 0,00448 |
| L-Phénylalanine | 0,00496 |
| L-Proline | 0,03453 |
| L-Serine | 0,06306 |
| L-Thréonine | 0,01191 |
| L-Tryptophane | 0,00306 |
| L-Tyrosine•2Na | 0,00341 |
| L-Valine | 0,03513 |
| D-Biotine | 0,0000146 |
| Chlorure de Choline | 0,01396 |
| Acide folique | 0,00079 |
| myo-Inositol | 0,01802 |
| Niacinamide | 0,00003663 |
| D-Pantothenic Acid (hemicalcium) | 0,000238 |
| Pyridoxine•HCl | 0,00006171 |
| Riboflavine | 0,0000376 |
| Thiamine•HCl | 0,000337 |
| Vitamine B-12 | 0,000407 |
| Adénine•HCl | 0,03088 |
| D-Glucose | 1,081 |
| HEPES | 6,6 |
| Phénol Red•Na | 0,001242 |
| Putrescine•2HCl | 0,000161 |
| Acide Pyruvique •Na | 0,055 |
| Acide Thioctic | 0,000206 |
| Thymidine | 0,000727 |

Il peut s'agir également d'un milieu disponible dans le commerce modifié, par exemple le milieu MCDB153 comprenant en outre des acides aminés complémentaires, par exemple de la tyrosine, de la méthionine ou un mélange de ceux-ci, des sels inorganiques additionnels, par exemple du bicarbonate de sodium (NaHCO₃).

Dans la présente, le milieu M3 peut comprendre en outre au moins un complément choisi parmi l'extrait pituitaire bovin (BPE), l'insuline, la pénicilline streptomycine (PS), l'hydrocorticosone, le sérum de cheval, le sérum de veau, du facteur de croissance basique des fibroblastes (bFGF), du facteur de stimulation des Granulocyte Macrophage (« Granulocyte Macrophage Colony Stimulating Factor » (GM-CSF)), du SCF ou un quelconque mélange de ceux-ci.

Dans la présente, le milieu M3 peut comprendre de 0,1 à 10%, en poids, de 0,5 à 5 % en poids, 1% en poids de pénicilline streptomycine (PS) par rapport au poids total du milieu.

Dans la présente, le milieu M3 peut comprendre une concentration de 1,25 à 1,60 µM, de 1,40 à 1,55 µM, de 1,45 µM d'hydrocortisone.

Dans la présente, le milieu M3 peut comprendre une concentration de 100 à 160 µg.mL⁻¹, de 110 à150 µg.mL⁻¹, égale à 140 µg.mL⁻¹ d'extrait pituitaire bovin (BPE).

Dans la présente, le milieu M3 peut comprendre une concentration de 15 à 25 µg.mL⁻¹, égale à 20 µg.mL⁻¹ d'insuline

Dans la présente, le milieu M3 peut comprendre une concentration de 0,01 à 0,2 µg.mL⁻¹, de 0,01 à 0,1 µg.mL⁻¹, égale à 0,01 µg.mL⁻¹ de GM-CSF.

Dans la présente, le milieu M3 peut comprendre une concentration de 0,004 à 0,2 µg.mL⁻¹, de 0,01 à 0,15 µg.mL⁻¹, égale à 0,05 µg.mL⁻¹ de SCF.

Dans la présente, le milieu M3 peut comprendre une concentration de 0,1 à 10 ng.mL⁻¹, de 0 ,5 à 5 ng.mL⁻¹, de 0,8 à 2 ng.mL⁻¹, égale à 1 ng.mL⁻¹ de bFGF.

Dans la présente, le milieu M3 peut comprendre de 1 à 5% en poids, de 2 à 4% en poids, 3% en poids de sérum de cheval ou de veau par rapport au poids total du milieu.

Dans la présente le milieu M3 et/ou l'ensemble de ses constituants peu(ven)t être de grade clinique.

Dans la présente, l'étape d. de culture de mélanocytes peut être réalisée à une température ambiante, par exemple à une température de 30 à 40°C, par exemple égale à 37°C.

Dans la présente, la durée de culture de l'étape d. peut être comprise entre 15 à 28 jours.

Dans la présente, la culture de l'étape d. de mélanocytes peut être réalisée sous une atmosphère contrôlée comprenant au moins 5 % de CO₂.

Selon l'invention, les mélanocytes obtenus par culture selon l'étape d. peuvent former un tapis de cellules à confluence dans le contenant de culture. Par exemple, les mélanocytes peuvent former un tapis de cellules de 50 à 100% de confluence.

Selon l'invention, lorsque la culture selon l'étape d. correspond à un tapis de cellule à confluence ou non, le procédé peut comprendre en outre :
- une étape d' d'élimination du milieu de culture, rinçage des cellules par une solution, élimination de la solution de rinçage,
- étape d" de détachement des cellules par trypsinisation, et
- une étape d'" de culotage.

Dans la présente, dans l'étape d', l'élimination du milieu de culture peut être réalisée par tout procédé connu de l'homme du métier adapté. Il peut s'agir par exemple d'une aspiration du milieu, d'un retournement du contenant afin d'éliminer le milieu de culture.

Dans la présente, dans l'étape d', le rinçage des cellules peut être réalisé par tout procédé connu de l'homme du métier, par exemple, par aspersion, trempage des cellules dans une solution de rinçage.

Dans la présente, par solution de rinçage de mélanocytes on entend toute solution de rinçage de mélanocytes connue de l'homme du métier. Il peut s'agir par exemple d'une solution tampon HBSS, par exemple de la solution décrite dans le tableau 2 ci-dessus, de tampon phosphate salin (PBS) à pH compris de 7,2 à 7,4. Il peut s'agir également d'une solution tampon disponible dans le commerce par exemple un tampon phosphate salin (PBS), une solution équilibrée de Hank commercialisée respectivement par la société Gibco, Sigma Aldrich, Lonza.

Dans la présente, dans l'étape d' l'élimination la solution de rinçage peut être réalisée par tout procédé connu de l'homme du métier adapté. Il peut s'agir par exemple d'une aspiration de la solution de rinçage, d'un retournement du contenant afin d'éliminer la solution de rinçage.

Selon l'invention, l'étape d" de trypsinisation peut être réalisée par immersion de cellules dans une solution tampon (ST) comprenant de la trypsine, suivie de l'ajout de Sérum de Veau Fœtal (SVF) afin d'arrêter la réaction enzymatique.

Selon l'invention, la solution tampon (ST) peut être une solution tampon telle que définie précédemment.

Selon l'invention la quantité de trypsine ajoutée dans la solution tampon (ST) peut être de 0,01 à 0,05 % en poids par rapport au poids total.

Selon l'invention la durée d'incubation de la trypsine avant ajout du SVF dans la solution tampon peut être de 2 à 5 min.

Dans la présente la quantité de SVF ajoutée dans la solution (ST) peut être comprise de 5 à 20 % en volume par rapport au volume total.

Selon l'invention, l'étape d'" de culottage peut être réalisée par sédimentation, par centrifugation par tout procédé connu de l'homme du métier. Il peut s'agir par exemple d'une centrifugation à une vitesse de 800 à 1200 tours par minutes.

Selon l'invention, l'étape d'" de centrifugation peut être réalisée pendant une durée de 5 à 10 min.

Dans la présente, l'étape d'" de centrifugation peut être réalisée par tout dispositif connu de l'homme du métier. Il peut s'agir par exemple d'une centrifugeuse rotative commercialisée par les sociétés Eppendorf ou Jouan.

Dans la présente, l'étape d'" de centrifugation permet de sédimenter les cellules afin de les séparer du milieu. L'homme du métier, de par ses connaissances générales, saura adapter/modifier l'étape d'" de centrifugation par toute technique connue permettant de sédimenter des cellules dans un milieu.

Selon l'invention, l'étape e. de culture de kératinocytes peut être réalisée dans tout contenant de culture adapté connu de l'homme du métier. Il peut s'agir d'une boite de pétri, d'un flacon de culture d'une contenance de 25 à 75 cm², de 25, 75 ou 125 cm².

Dans la présente le milieu M4 de culture de kératinocytes peut être tout milieu connu de l'homme du métier adapté à la culture de kératinocytes. Il peut s'agir par exemple d'un milieu disponible dans le commerce, par exemple un milieu KSFM commercialisé par la société life-technology, KGM commercialisé par la société lonza, provitro dans un milieu MCDB 153 commercialisé par la société Sigma-Aldrich comprenant notamment un mélange d'acides aminés, de vitamines, de sels inorganiques de sucres, par exemple du glucose. Il peut s'agir également d'un milieu disponible dans le commerce modifié, par exemple le milieu MCDB153 comprenant une concentration de chlorure de sodium de 0,100 à 0,110 M/L, par exemple de 0,104M/L, une concentration d'Hepes de 2 à 3x10⁻²M/L, par exemple de 2,29 x10⁻²M/L, une concentration de bicarbonate de sodium de 1,10 x10⁻²M/L à 1,25 x10⁻²M/L, par exemple de 1,19 x10⁻²M/L, et comprenant une concentration en arginine, en histidine, en isoleucine, en leucine, en méthionine, en phénylalanine, en thréonine, en tryptophane, en tyrosine, en valine et en choline doublée par rapport aux concentrations du milieu MCDB153 non modifié.

Dans la présente, le milieu M4 peut comprendre en outre des compléments choisis parmi des facteurs de croissance, par exemple du facteur de croissance épithélial (EGF), d'extrait pituitaire bovin (BPE), d'insuline, de pénicilline- streptomycine (PS), d'hydrocorticosone ou un quelconque mélange de ceux-ci. Avantageusement, le milieu M4 peut comprendre des compléments de grade clinique. Il peut s'agir par exemple de compléments choisis parmi des facteurs de croissance, par exemple du facteur de croissance épithélial (EGF), d'insuline, de pénicilline-streptomycine (PS), d'hydrocorticosone ou un quelconque mélange de ceux-ci.

Dans la présente, le milieu M4 peut comprendre par exemple de 0,5 à 5% en poids, de 0,75 à 3% en poids, 1% en poids de pénicilline-streptomycine (PS) par rapport au poids total du milieu.

Dans la présente, le milieu M4 peut comprendre une concentration de 1,25 à 1,60µM, de 1,40 à 1,55µM, de 1,45µM d'hydrocortisone.

Dans la présente, le milieu M4 peut comprendre une concentration de 50 à 90 µg.mL₋₁, de 60 à 80 µg.mL⁻¹, de 70 µg.mL⁻¹ d'extrait pituitaire bovin (BPE).

Dans la présente, le milieu M4 peut comprendre une concentration de 3 à 8 µg.mL⁻¹, par exemple égale à 5 µg.mL⁻¹ d'insuline.

Dans la présente, le milieu M4 peut comprendre une concentration de 5 à 15 ng.mL⁻¹, de 6,5 à 13 ng.mL⁻¹, égale à 10 ng.mL⁻¹ de facteur de croissance épithélial (EGF).

Dans la présente le milieu M4 et/ou l'ensemble de ses constituants peu(ven)t être de grade clinique.

Dans la présente, l'étape e. de culture de kératinocytes peut être réalisée à une température de 25 à 39°C, par exemple égale à 37°C.

Dans la présente, la durée de culture de l'étape e. peut être comprise de 15 à 28 jours.

Dans la présente, la culture de l'étape e. de kératinocytes peut être réalisée sous une atmosphère contrôlée comprenant au moins 5 % de CO₂.

Selon l'invention, les kératinocytes obtenus par culture selon l'étape e. peuvent former une monocouche de cellules dans le contenant de culture. Il peut s'agir par exemple d'une monocouche de cellules proche de la confluence, par exemple de 50 à 80 % de confluence dans le contenant de culture.

Selon l'invention, lorsque la culture selon l'étape e. correspond à une monocouche de cellules proche de la confluence, de préférence de 50 à 80 % de confluence, le procédé peut comprendre en outre :
- une étape e' d'élimination du milieu de culture, rinçage des cellules par une solution, élimination de la solution de rinçage,
- une étape e" de détachement des cellules par trypsinisation, et
- une étape e'" de centrifugation.

Dans la présente, dans l'étape e' l'élimination du milieu de culture peut être réalisée par tout procédé connu de l'homme du métier adapté. Il peut s'agir par exemple d'une aspiration du milieu, d'un renversement du contenant afin d'éliminer le milieu de culture.

Dans la présente, dans l'étape e' le rinçage des cellules peut être réalisé par tout procédé connu de l'homme du métier, par exemple, par trempage, aspersion, incubation des cellules dans une solution de rinçage de kératinocytes.

Dans la présente, par solution de rinçage de kératinocytes on entend toute solution de rinçage de kératinocytes connue de l'homme du métier. Il peut s'agir par exemple d'une solution tampon PBS, HBSS, par exemple tel que décrit dans le tableau 2 ci-dessus, à pH compris de 7,2 à 7,4. Il peut s'agir également d'une solution tampon disponible dans le commerce, par exemple un tampon phosphate salin (PBS), une solution équilibrée de Hank's commercialisée respectivement par la société Gibco, Sigma Aldrich, Lonza.

Dans la présente, dans l'étape e' l'élimination de la solution de rinçage de kératinocytes peut être réalisée par tout procédé connu de l'homme du métier adapté. Il peut s'agir par exemple d'une aspiration de la solution de rinçage de kératinocytes, d'un retournement du contenant afin d'éliminer la solution de rinçage de kératinocytes.

Selon l'invention, l'étape e" de trypsinisation peut être réalisée par immersion des cellules dans une solution (S) comprenant de trypsine, suivi de l'ajout de Sérum de Veau Fœtal (SVF) afin d'arrêter la réaction enzymatique.

Selon l'invention la quantité de trypsine ajoutée dans la solution (S) peut être comprise de 0,01 à 0,05 % en poids par rapport au poids total de la solution.

Selon l'invention la durée d'incubation de la trypsine avant ajout du SVF dans le milieu peut être comprise de 5 à 10 min.

Dans la présente la quantité de SVF ajoutée dans la solution (S) peut être comprise de 5 à 20 % en poids par rapport au poids total de la solution.

Selon l'invention, l'étape e'" de centrifugation peut être réalisée par tout procédé connu de l'homme du métier. Il peut s'agir par exemple d'une centrifugation à une vitesse de 800 à 1200 tours par minutes.

Selon l'invention, l'étape e'" de centrifugation peut être réalisée pendant une durée de 5 à 10 min.

Dans la présente, l'étape e'" de centrifugation peut être réalisée par tout dispositif connu de l'homme du métier. Il peut s'agir par exemple d'une centrifugeuse rotative commercialisée par les sociétés Eppendorf ou Jouan.

Dans la présente l'étape f. de mélange de mélanocytes obtenus à l'étape d. avec des kératinocytes obtenus à l'étape e. peut être réalisée par tout moyen procédé adapté connu de l'homme du métier. Il peut s'agir par exemple d'un mélange de cellules sous agitation dans un milieu de culture.

Dans la présente, le mélange de mélanocytes et de kératinocytes de l'étape f peut être réalisé avec un ratio mélanocytes / kératinocytes en nombre de 1/20 à 1/15, égal à 1/19.

Avantageusement, les inventeurs ont démontré de manière surprenante que lorsque le mélange de mélanocytes et de kératinocytes est réalisé avec un ratio mélanocytes / kératinocytes de 1/20 à 1/15, de préférence égale à 1/19, le substitut de peau obtenu présente des caractéristiques structurelles/biologiques identiques à celles d'une peau in-vivo.

Dans un mode de réalisation préféré, le mélange de mélanocytes et de kératinocytes de l'étape f. est réalisé avec un ratio mélanocytes / kératinocytes en nombre de 1/20 à 1/15, égal à 1/19, et la matrice comprenant du collagène est une matrice de régénération dermique telle que définie ci-avant.

Dans la présente, l'ensemencement du substitut de derme de l'étape g peut être réalisé par tout procédé connu de l'homme du métier. Il peut s'agir par exemple d'une application, par exemple par aspersion du milieu de culture comprenant un mélange de mélanocytes et de kératinocytes obtenus à l'étape f., par dépôt par repiquage des cellules sur le substitut de derme, par versement au goutte à goutte du milieu de culture comprenant un mélange de mélanocytes et de kératinocytes obtenus à l'étape f., par impression 3D par exemple tel que décrit dans Wonhye Lee et al. « Multi-layered culture of human skin fibroblasts and keratinocytes through three-dimensional freeform fabrication." Biomaterials, 2009, Mar;30(8):1587-95 [9].

Dans la présente, l'ensemencement du substitut de derme de l'étape g peut être avantageusement réalisé avec un ratio (kératinocytes + mélanocytes)/fibroblastes de 9 à 19. Les inventeurs ont en effet démontré de manière surprenante que lorsque l'ensemencement de l'étape g est réalisé avec un ratio (kératinocytes + mélanocytes)/fibroblastes de 9 à 19, le substitut de peau obtenu présente des caractéristiques structurelles/biologiques identiques à celles d'une peau normale.

Dans un mode de réalisation préféré, l'ensemencement du substitut de derme de l'étape g est réalisé avec un ratio (kératinocytes + mélanocytes)/fibroblastes de 9 à 19 et la matrice comprenant du collagène est une matrice de régénération dermique telle que définie ci-avant.

Dans la présente par milieu M5 de culture de peau on entend tout milieu connu de l'homme du métier adapté à la culture de peau. Il peut s'agir par exemple d'un milieu disponible dans le commerce, par exemple un milieu Green modifié à savoir comprenant 2/3 de milieu « Dulbecco/Vogt modified Eagle's minimal essential médium » (DMEM); 1/3 de milieu « Ham's F12 » et comprenant 10% de Sérum de Veau Fœtal (SVF) mélange à façon commercialisé par la société Gibco comprenant notamment un mélange d'acides aminés, de vitamines, de sels inorganiques, de sucres, par exemple du glucose. Il peut s'agir également d'un milieu Green modifié, c'est-à-dire un milieu Green exempt de choléra toxine, de triodothyronine, ou d'un mélange du milieu « Iscove's Modified Dulbecco's Médium » (IMDM) et du milieu MCDB153 comprenant 10% de SVF ; ou d'un mélange IMDM / « dermalife keratinocyte » comprenant 10% de SVF commercialisé respectivement par la société Gibco, Lifescience, Promocell et Sigma Aldrich.

Dans la présente, le milieu M5 peut également comprendre en outre des compléments choisis parmi l'acide hyaluronique ou un hyaluronate ou un dérivé de ceux-ci, l'acide ascorbique ou un ascorbate ou un dérivé de ceux-ci, ou un mélange de ceux-ci.

Dans la présente, le milieu M5 peut comprendre par exemple de 40 à 60 mg.L⁻¹, de 45 à 55 mg.L⁻¹, 50 mg.L⁻¹ de hyaluronate ou acide hyaluronique.

Avantageusement, lorsque le milieu M5 comprend de l'acide hyaluronique ou un hyaluronate et/ou un dérivé de ceux-ci il ne comprend pas d'extrait pituitaire de bovin.

Dans la présente, le milieu M5 peut comprendre par exemple de 40 à 60 mg.L⁻¹, de 45 à 55 mg.L⁻¹, 50 mg.L⁻¹ d'acide ascorbique ou ascorbate.

Dans la présente, l'étape h. de culture de peau peut être réalisée à une température comprise de 25 à 40°C, par exemple égale à 37°C.

Dans la présente, la durée de culture de peau de l'étape h. peut être comprise entre 6 et 21 jours, par exemple de 8 à 15 jours.

Dans la présente, la culture de peau de l'étape h. de peau peut être réalisée sous une atmosphère contrôlée comprenant au moins 5 % de CO₂.

Selon l'invention, la culture de peau de l'étape h. de peau peut être réalisée à une température comprise de 25 à 40°C, par exemple égale à 37°C et sous une atmosphère contrôlée comprenant au moins 5% de CO₂.

Dans la présente le milieu M5 et/ou chacun de ses composants peu(ven)t être de grade clinique.

Dans la présente l'étape h. peut comprendre :
- une première étape h.' de culture d'au moins 6 heures, de préférence de 6 à 24 heures, en présence d'un milieu M5¹ de culture ne comprenant ni acide hyaluronique, ni hyaluronate, ni acide ascorbique ni ascorbate,
- une deuxième étape h." de culture de 0 à 7 jours, de préférence d'au moins 2 jours, en présence d'un milieu M5² de culture comprenant de l'acide hyaluronique ou un hyaluronate ou un dérivé de ceux-ci, et
- une troisième étape h.'" de culture d'au moins 2 jours dans un milieu M5³ comprenant de l'acide hyaluronique ou un hyaluronate ou un dérivé de ceux-ci, et de l'acide ascorbique ou un ascorbate ou un dérivé de ceux-ci.

Le milieu M5¹ de culture de substitut de peau correspond au milieu M5 tel que défini ci-dessus ne comprenant ni acide ascorbique ni ascorbate ou dérivé de ceux-ci.

Dans la présente le milieu M5¹ et/ou chacun de ses composants peu(ven)t être de grade clinique.

Le milieu M5² de culture de substitut de peau correspond au milieu M5 tel que défini ci-dessus comprenant de l'acide hyaluronique ou un hyaluronate ou un dérivé de ceux-ci tout en étant exempt d'acide ascorbique, d'ascorbate et d'un dérivé de ceux-ci.

Dans la présente le milieu M5² peut être et/ou chacun de ses composants peu(ven)t être de grade clinique.

Le milieu M5³ de culture de substitut de peau correspond au milieu M5 tel que défini ci-dessus comprenant de l'acide hyaluronique ou un hyaluronate ou un dérivé de ceux-ci et de l'acide ascorbique ou de l'ascorbate ou un dérivé de ceux-ci.

Dans la présente le milieu M5³ peut être un milieu de grade clinique.

Dans la présente invention, l'étape h' de culture peut être réalisée par dépôt dans un milieu de culture M5¹ du substitut de derme ensemencé obtenu à l'étape g.

Dans la présente, l'étape h'. de culture peut être réalisée à une température comprise de 25 à 40°C, par exemple égale à 37°C.

Dans la présente, la durée de l'étape h'. de culture peut être de 6 à 24 heures, par exemple de 8 à 24 heures, de 12 à 18 heures.

Dans la présente, l'étape h'. de culture de peau peut être réalisée sous une atmosphère contrôlée comprenant au moins 5 % de CO₂.

Avantageusement, les inventeurs ont démontré que l'étape h' permet de favoriser l'adhésion des mélanocytes et kératinocytes sur le substitut de derme.

Dans la présente invention, l'étape h". de culture peut être réalisée par dépôt dans un milieu de culture M5² du substitut de derme ensemencé obtenu à l'étape h'. ou immersion ou submersion du substitut de derme ensemencé obtenu à l'étape h' dans un milieu de culture M5².

Dans la présente, l'étape h". de culture peut être réalisée à une température comprise de 25 à 40 °C, par exemple égale à 37°C.

Dans la présente, la durée de l'étape h". de culture peut être comprise de 0 à 7 jours, de préférence de 2 à 7 jours.

Dans la présente, l'étape h". de culture de peau peut être réalisée sous une atmosphère contrôlée comprenant au moins 5.% de CO₂.

Dans la présente invention, l'étape h'", de culture peut être réalisée par dépôt dans un milieu de culture M5² du substitut de derme ensemencé obtenu à l'étape h"., immersion du substitut de derme ensemencé obtenu à l'étape h" dans un milieu de culture M5², ou immersion du substitut de derme ensemencé obtenu à l'étape h"., ledit substitut étant immergé dans ledit milieu jusqu'à l'interface air-liquide, ou ledit milieu étant immergé à fleur de milieu.

Dans la présente par « à fleur de milieu » on entend l'immersion du substitut dans le milieu de manière à recouvrir le substitut sur la totalité de sa hauteur sans immersion de sa partie supérieure.

Dans la présente, l'étape h'", de culture peut être réalisée à une température comprise de 25 à 40°C, par exemple égale à 37°C.

Dans la présente, la durée de l'étape h'", de culture peut être comprise de 2 à 7 jours de préférence 7 jours.

Dans la présente, l'étape h'", de culture de peau peut être réalisée sous une atmosphère contrôlée comprenant au moins 5% de CO₂

Avantageusement, les inventeurs ont démontré que l'immersion du substitut de derme ensemencé obtenu à l'étape h". dans un milieu de culture M5³ comprenant de l'acide hyaluronique ou un hyaluronate ou un dérivé de ceux-ci et de l'acide ascorbique ou un ascorbate ou un dérivé de ceux-ci permet de favoriser la formation de la jonction dermo-épidermique et assure ainsi une meilleure polarisation des cellules du substitut de derme ensemencé.

Avantageusement, les inventeurs ont également démontré que l'immersion jusqu'à l'interface air-liquide ou à fleur de milieu du substitut de derme ensemencé obtenu à l'étape h". dans un milieu de culture M5³ comprenant de l'acide hyaluronique ou un hyaluronate ou un dérivé de ceux-ci et de l'acide ascorbique ou un ascorbate ou un dérivé de ceux-ci permet une différenciation de l'épiderme favorisant ainsi la formation d'une couche cornée sur le substitut ou équivalent de peau.

Avantageusement, les inventeurs ont également démontré que l'immersion jusqu'à l'interface air-liquide ou à fleur de milieu du substitut de derme ensemencé obtenu à l'étape h" dans un milieu de culture M5³ comprenant de l'acide hyaluronique ou un hyaluronate ou un dérivé de ceux-ci et de l'acide ascorbique ou un ascorbate ou un dérivé de ceux-ci permet un maintien du haut pouvoir prolifératif des cellules de la couche basale et un gradient décroissant de pouvoir prolifératif concomitant à l'augmentation de la différenciation de l'épiderme similaire aux gradients observés dans la peau in vivo.

Avantageusement, les inventeurs ont également démontré que lorsque le milieu M5 comprend de l'acide hyaluronique ou un hyaluronate ou un dérivé de ceux-ci, cela permet, de manière surprenante, d'améliorer la qualité du substitut ou équivalent de peau.

Avantageusement, les inventeurs ont démontré que le procédé permet avantageusement d'obtenir un substitut de peau ou équivalent de peau comprenant l'ensemble des couches constitutives de celles-ci. Aussi, le substitut de peau obtenu selon le procédé de l'invention présente des caractéristiques similaires à celle de la peau native au contraire des substituts de peau connus dans l'état de la technique.

Avantageusement, les inventeurs ont également démontré que le procédé permet d'obtenir un substitut de derme et/ou un substitut de peau d'une taille bien supérieure à ceux connus dans l'état de la technique. En particulier, le procédé permet avantageusement d'obtenir un substitut de derme et/ou un substitut de peau avec une surface de 1 à 25 cm², par exemple de 5 à 25 cm².

Les inventeurs ont également avantageusement démontré que le procédé permet d'obtenir un substitut ou équivalent de derme et/ou un équivalent ou substitut de peau avec une capacité d'amplification minimale de 6

En outre, les inventeurs ont également avantageusement démontré que le procédé selon l'invention permet d'obtenir un substitut de derme et/ou de peau manipulable avec des propriétés viscoélastiques permettant avantageusement d'éviter une éventuelle déchirure/altération physique dudit substitut lors de sa manipulation.

En outre, la durée de préparation du substitut de derme et/ou de peau selon le procédé de l'invention est typiquement inférieure à 30 jours, et est donc compatible avec les exigences pour le soin des brûlés notamment.

La présente invention a également pour objet un substitut de peau susceptible d'être obtenu par la mise en œuvre du procédé tel que défini ci-dessus.

Avantageusement, les inventeurs ont démontré que le substitut de peau comprend des mélanocytes au niveau de l'assise basale formant une unité épidermique de mélanisation.

Avantageusement, le substitut de peau susceptible d'être obtenu par le procédé de l'invention, de par la présence de mélanocytes présente avantageusement une pigmentation constitutive.

Avantageusement, le substitut de peau a une taille bien supérieure à ceux connus dans l'état de la technique permettant notamment lors de plaies étendues, l'application d'un seul ou d'un nombre inférieur de substituts de peau par rapport à ceux de l'état de la technique. La diminution du nombre de substituts de peau à appliquer permet également avantageusement de réduire les frais de traitement tout en accélérant ce dernier.

La présente invention a également pour objet un substitut de derme susceptible d'être obtenu à l'étape c. du procédé.

Avantageusement, le substitut de derme peut comprendre du collagène de type IV néoformé.

Avantageusement le substitut de derme comporte au moins une matrice comprenant du collagène de type I dans laquelle sont répartis les fibroblastes. Elle peut contenir en outre d'autres constituants de la matrice extracellulaire, par exemple des molécules telles que les collagènes en particulier le collagène IV, les laminines, les glycosaminoglycanes.

Avantageusement, le substitut de derme a une taille bien supérieure à ceux connus dans l'état de la technique permettant notamment lors de plaies étendues, l'application d'un seul ou d'un nombre inférieur de substituts par rapport à ceux de l'état de la technique. La diminution du nombre de substituts à appliquer permet également avantageusement de réduire les frais de traitement tout en accélérant le traitement de part, par exemple la mise à disposition d'un dispositif unique couvrant l'ensemble de la surface à traiter.

Les inventeurs ont également démontré que le substitut de derme et/ou le substitut de peau selon l'invention peut être avantageusement utilisé pour la couverture de pertes de substance, le substitut de derme et/ou le substitut de peau selon l'invention peut donc être avantageusement utilisé comme greffon.

La présente invention a donc également pour objet un greffon constitué d'un substitut de peau tel que défini ci-dessus ou substitut de derme tel que défini ci-dessus.

Dans la présente, le greffon selon l'invention peut être utilisé comme moyen de traitement d'un désordre cutané et/ou d'une perte de substance cutanée. En particulier, le greffon selon l'invention peut être utilisé comme moyen de traitement d'un désordre cutané et/ou d'une perte de substance cutanée choisie dans le groupe comprenant une brûlure, un défaut de cicatrisation, lié à une plaie traumatique ou à une plaie chronique, un désordre pigmentaire, un hémangiome et un cancer cutané.

La présente invention a donc également pour objet un procédé de traitement d'un trouble de la peau comprenant la transplantation ou l'implantation d'un substitut de peau ou d'un substitut de derme selon l'invention.

Dans la présente, l'implantation du substitut de peau peut être réalisée par tout procédé connu de l'homme du métier. Il peut s'agir par exemple de l'application directe du substitut sur la zone à traiter, par exemple selon le procédé décrit dans Peña, and al. Use of Autologous Skin Equivalents With Artificial Dermal Matrix (Integra) in Donor Site Coverage in Radial Forearm Free Flaps: Preliminary Cases J Oral and Maxillofacial Surgery, 70:10 10, 2012 [10]

Dans la présente, la transplantation du substitut de peau peut être réalisée par tout procédé connu de l'homme du métier. Il peut s'agir par exemple d'un procédé comprenant une première étape de détermination et d'élimination d'une zone de peau/derme à éliminer suivie d'une seconde étape d'incorporation du substitut dans la zone laissée libre. Il peut s'agir également du procédé décrit dans le document E.Dantzer, F. Braye Reconstructive surgery using an artificial dermis (Integra): results with 39 grafts. Br J Plast Surg, 54:8 8, 2001 [11].

Dans le procédé de traitement, par trouble de la peau on entend une brûlure, un défaut de cicatrisation lié à une plaie traumatique ou à une plaie chronique, un désordre pigmentaire, un hémangiome et un cancer cutané.

Le procédé de traitement peut donc permettre le remplacement d'une peau lésée et/ou endommagée et/ou pathologique par un substitut de peau sain.

Le procédé de traitement peut comprendre la transplantation du substitut de peau après ablation, par exemple d'un nævus mélanocytaire, d'un nævus géant mélanocytaire, d'un mélanome, d'un hémangiome, d'un porome eccrine.

Le procédé de traitement peut également comprendre l'implantation et/ou l'application d'un substitut de peau sur une brûlure et/ou une blessure profonde et/ou une plaie chronique, par exemple une plaie diabétique.

Avantageusement, les inventeurs ont démontré de manière surprenante, lorsque le substitut ou équivalent de peau a été obtenu selon le procédé de l'invention dans lequel le milieu M5 comprend de l'acide hyaluronique ou un hyaluronate ou un dérivé de ceux-ci, une accélération de la réparation tissulaire notamment lors de traitement d'un trouble de la peau.

La présente invention a également pour objet un kit pour la mise en œuvre du procédé selon l'invention comprenant un milieu M1 de culture de fibroblastes, une matrice comprenant du collagène, un milieu M2 de culture des fibroblastes dans la matrice comprenant de l'acide ascorbique ou ascorbate ou dérivé de ceux-ci, un milieu M3 de culture de mélanocytes, un milieu M4 de culture de kératinocytes et un milieu M5 de culture de peau.

La présente invention a également pour objet un kit pour la mise en œuvre du procédé selon l'invention comprenant un milieu M1 de culture de fibroblastes, une matrice comprenant du collagène, un milieu M2¹, un milieu M2² de culture des fibroblastes dans la matrice comprenant du collagène, un milieu M3 de culture de mélanocytes, un milieu M4 de culture de kératinocytes, un milieu M5¹ et/ou un milieu M5² et/ou un milieu M5³ de culture de peau.

Les milieux M1, M2, M2¹, M2², M3, M4, M5, M5¹, M5² et M5³ sont tels que définis ci-dessus.

La présente invention a également pour objet un procédé de greffe de peau comprenant les étapes de :
- prélèvement d'un échantillon de peau au niveau d'une zone non affectée chez un individu,
- préparation d'un substitut de peau selon l'invention en utilisant les fibroblastes, kératinocytes et fibroblastes issus de l'échantillon prélevé, et
- la greffe du substitut obtenu sur l'individu.

D'autres avantages pourront encore apparaître à l'homme du métier à la lecture des exemples ci-dessous, illustrés par les figures annexées, donnés à titre illustratif.

### Brève description des figures

La figure 1 représente un schéma des étapes permettant l'obtention d'un substitut/équivalent de peau.
La figure 2 représente des photographies en microscopie optique de peau (figure 2A), de substituts de peau obtenus selon le procédé de l'invention avec des variations de ratio de cellules ensemencées (figures 2B et 2C).

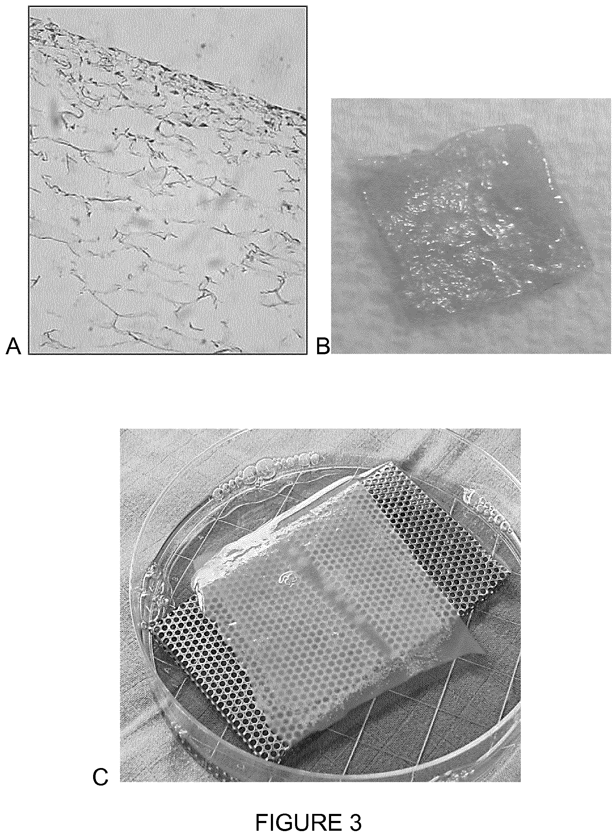

La figure 3 A est une photographie d'un substitut de derme obtenu à l'étape c en microscopie optique après coloration des fibroblastes. La figure 3B est une photographie d'un substitut de peau obtenu de petite taille à savoir 0,5 cm². La figure 3C est une photographie d'un substitut de peau obtenu de taille moyenne à savoir 25 cm².
La figure 4 représente des photographies après greffe d'une matrice comprenant du collagène (colonne A) ou d'un substitut de derme obtenu à l'étape c (colonne B) au jour de greffe (ligne 1), 14 jours après greffe (ligne 2) ou 24 jours après greffe (ligne 3).
La figure 5 représente des photographies en microscopie optique d'un prélèvement du greffon 24 jours après greffe après coloration hematoxyline-eosine

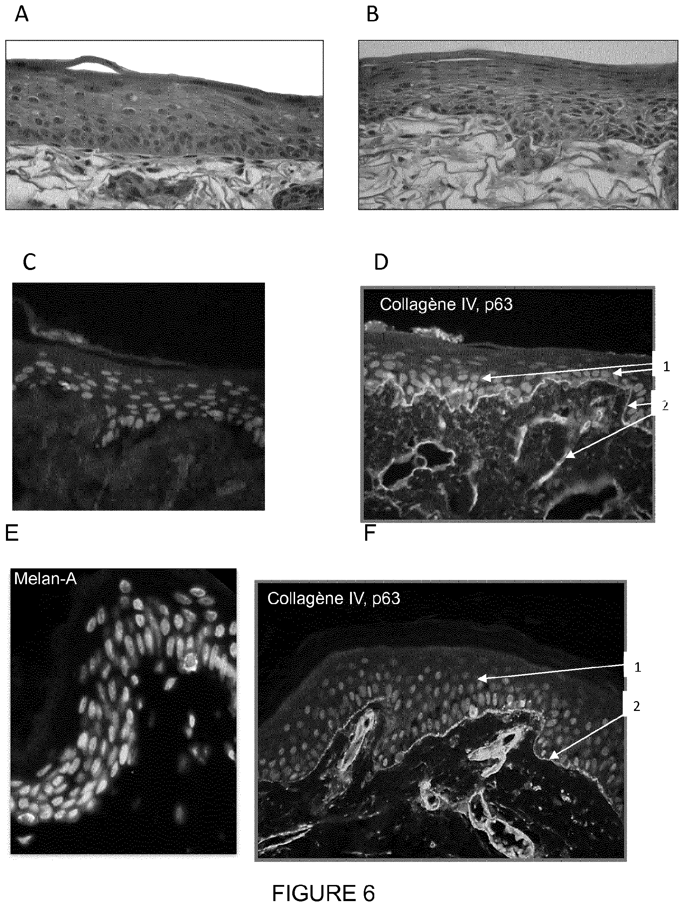

La figure 6 représente des photographies en microscopie optique d'équivalent de peau (figure 6 A à D) obtenu selon le procédé avec un ratio de cellules ensemencées (kératinocytes + mélanocytes)/fibroblastes de 13,3 (Figures 6 A et B) ; le marquage immunohistochimique des mélanocytes en position basale (figure 6 C, zones claires) et la production de la lame basale, marquage collagène IV (figure 6D (2)) et de la p63 marqueur de prolifération (figure 6 D (1)). Les figures 6E et 6F représentent des photographies en microscopie optique de peau après le marquage immunohistochimique des mélanocytes en position basale (figure 6E, zones claires), le marquage du collagène IV (figure 6F (2)) et de la p63 marqueur de prolifération (figure 6F (1)).

### EXEMPLES

### Exemple 1 : Exemple de fabrication d'un substitut de peau

Dans le présent exemple, les cellules utilisées provenaient d'une biopsie de peau effectuée au niveau de plasties mammaires préalablement mises en œuvre sur un patient

Le prélèvement de la biopsie a été réalisé par un chirurgien plasticien et la biopsie déposée dans un tube stérile contenant du sérum physiologique.

A partir de la biopsie, les cellules ont été isolées comme suit :

### 1. Isolement des cellules épithéliales

**a.** Rinçage de la biopsie dans du HBSS (Hank's Balanced Salt Solution) stérile
**b.** Enlèvement du tissu adipeux par un technicien biologiste à l'aide d'un scalpel
**c.** Incubation avec de la trypsine-EDTA préchauffée à 37 °C entre 3 et 24 h
**d.** Neutralisation avec SVF irradié (inhibiteur de trypsine)
**e.** Enlèvement de l'épiderme et grattage au scalpel de l'assise basale où se trouvent les cellules hautement prolifératives (p63 positives)
**f.** Filtration, centrifugation à 1200 tours par minutes et ensemencement du culot à 100 000 cellules par cm² dans du milieu MCDB153 modifié* comprenant les composés mentionnés dans le tableau 4 ci-dessous dans lequel les concentrations de la L arginine, la L Histidine, L isoleucine, L leucine, L méthionine , L phénylalanine, L thréonine, L- tryptophane, L tyrosine, L valine, Chlorure de Choline ont été doublées, la concentration de Nacl a été diminuée à 0,104 M/L, Hepes à 2,29 exp⁻² M/L et le NaHCO₃ à 1,19exp⁻² M/L, le pH du milieu étant ajusté à 7,4 et des antibiotiques (pénicilline et streptomycine 1 %) pour les kératinocytes.
Pour les mélanocytes, Filtration, centrifugation à 1200 tours par minute et ensemencement du culot à 100 000 cellules par cm² dans du milieu MCDB153 modifié* comprenant les composés mentionnés dans le tableau 4 ci-dessous comprenant en outre 5,88 g de sodium bicarbonate/5L, 0,272 g de tyrosine/5L et 0,157 g de L Méthionine/ 5L, le pH du milieu étant ajusté à 7,4

**Tableau 4 : composition normale du milieu MCDB 153**

| **Composition** | **Concentration en g.L⁻¹** |
|---|---|
| Ammonium Metavanadate | 0,000000585 |
| Chlorure de Calcium •Anhydre | 0,00333 |
| Cupric Sulfate•5H2O | 0,00000275 |
| Sulfate Ferreux•7H2O | 0,00139 |
| Chlorure de Magnésium | 0,05713 |
| Manganèse Sulfate | 0,000000151 |
| Molybdic Acide•4H2O (ammonium) | 0,00000124 |
| Nickel Chloride•6H2O | 0,00000012 |
| Potassium Chloride | 0,11183 |
| Sodium Acétate (anhydre) | 0,30153 |
| Chlorure de Sodium | 7,599 |
| Sodium Metasilicate•9H2O | 0,000142 |
| Sodium Phosphate Dibasique (anhydre) | 0,284088 |
| Sodium Sélénite | 0,0000038 |
| Stannous Chloride•2H2O | 0,000000113 |
| Zinc Sulfate•7H2O | 0,000144 |
| L-Alanine | 0,00891 |
| L-Arginine•HCl | 0,2107 |
| L-Asparaqine•H2O | 0,015 |
| L-Aspartique Acide | 0,00399 |
| L-Cysteine•HCl•H2O | 0,04204 |
| L-Glutamique Acide | 0,01471 |
| L-Glutamine | 0,8772 |
| Glycine | 0,00751 |
| L-Histidine•HCl•H2O | 0,01677 |
| L-Isoleucine | 0,001968 |
| L-Leucine | 0,0656 |
| L-Lysine•HCl | 0,01827 |
| L-Méthionine | 0,00448 |
| L-Phénylalanine | 0,00496 |
| L-Proline | 0,03453 |
| L-Serine | 0,06306 |
| L-Thréonine | 0,01191 |
| L-Tryptophane | 0,00306 |
| L-Tyrosine•2Na | 0,00341 |
| L-Valine | 0,03513 |
| D-Biotine | 0,0000146 |
| Chlorure de Choline | 0,01396 |
| Acide folique | 0,00079 |
| myo-Inositol | 0,01802 |
| Niacinamide | 0,00003663 |
| D-Pantothenic Acid (hemicalcium) | 0,000238 |
| Pyridoxine•HCl | 0,00006171 |
| Riboflavine | 0,0000376 |
| Thiamine•HCl | 0,000337 |
| Vitamine B-12 | 0,000407 |
| Adénine•HCl | 0,03088 |
| D-Glucose | 1,081 |
| HEPES | 6,6 |
| Phénol Red•Na | 0,001242 |
| Putrescine•2HCl | 0,000161 |
| Acide Pyruvique •Na | 0,055 |
| Acide Thioctic | 0,000206 |
| Thymidine | 0,000727 |

**g.** Incubation à 37°C à 5% CO₂ pendant une semaine avec changement du milieu tous les 3 jours.
**h.** Au bout d'une semaine environ : trypsinisation différentielle = trypsinisation avec trypsine 0,025 % et EDTA 0.0.1M (1-2 minutes pour décoller les mélanocytes, 10 minutes pour décoller les kératinocytes). Les mélanocytes se décollent en premier ce qui permet de purifier les cultures.
Neutralisation avec du SVF irradié, centrifugation à 1200 tours par minutes et ensemencement du culot pour amplification dans le même milieu.
**i.** Incubation à 37°C à 5% CO₂ pendant une semaine avec changement du milieu tous les 3 jours.

### 2. Isolement des fibroblastes

**a.** Rinçage de la partie dermique par HBSS,
**b.** Incubation du derme avec de la collagénase 1% à 37°C au maximum 3 heures selon le type de derme.
**c.** Neutralisation avec SVF irradié.
**d.** Filtration via un tamis cellulaire de 40 µm, centrifugation à 1200 tours minutes avec une centrifugeuse GR 2022 pendant 5 minutes et ensemencement du culot à 100 000 cellules par cm² dans du DMEM comprenant 10 % SVF irradié et de la pénicilline et de la streptomycine à 1% pendant 24 heures.
**e.** Incubation dans un incubateur Jouan IG 150 à 37°C 5% CO₂ pendant une semaine avec changement du milieu tous les 3 jours.

### 3. Préparation d'un substitut de peau

**a.** Trypsinisation des fibroblastes avec trypsine 0,025 % et EDTA 0.0.1M pendant 10 minutes puis neutralisation avec du SVF irradié, centrifugation à 1200 tours minutes avec une centrifugeuse GR 2022 pendant 5 minutes, et ensemencement dans du DMEM comprenant 10 %SVF sur une matrice dermique d'origine collagénique stérile, à savoir une matrice Integra (marque déposée) préalablement rincée avec une solution salée équilibrée de Hank (« Hank's Balanced Salt Solution » (HBSS) 3 fois à raison de 30 000 fibroblastes par cm² dans une chambre d'incubation en acier inoxydable faite sur mesure.
**b.** Au bout de 24 heures de culture à 37°C, 5 % CO₂, la chambre d'incubation a été enlevée de la matrice.
**c.** La matrice ensemencée a été incubée à 37°C, 5 % CO₂ dans du DMEM comprenant 10 % de SVF irradié et de la pénicilline et streptomycine à 1% et de l'acide ascorbique 50 mg/mL, pendant 1 semaine avec changement du milieu tous les 3 jours.
**d.** Trypsinisation des kératinocytes et des mélanocytes avec trypsine 0,025 % et EDTA 0.0.1M pendant 1 à 2 minutes pour décoller les mélanocytes des boites de culture mélanocytes, pendant 10 minutes pour décoller les kératinocytes des boites de culture kératinocytes. .Neutralisation avec du SVF irradié et centrifugation et ensemencement à 400 000 cellules par cm² dans une chambre d'incubation d'un mélange contenant 1 mélanocyte pour 19 kératinocytes.
**e.** Adhésion pendant 24 heures.
**f.** Submersion pendant 7 jours dans du milieu de green modifié :DMEM / Ham's F12/ 10% SVF comprenant de l'acide hyaluronique à 50 mg/mL.
**g.** Interface pendant 7 jours dans du milieu de green modifié DMEM /Ham's F12 comprenant du SVF à 10%, de l'acide hyaluronique à 50 mg/mL et de l'acide ascorbique 50 mg/mL et des antibiotiques à savoir à 1 % pénicilline streptomycine.

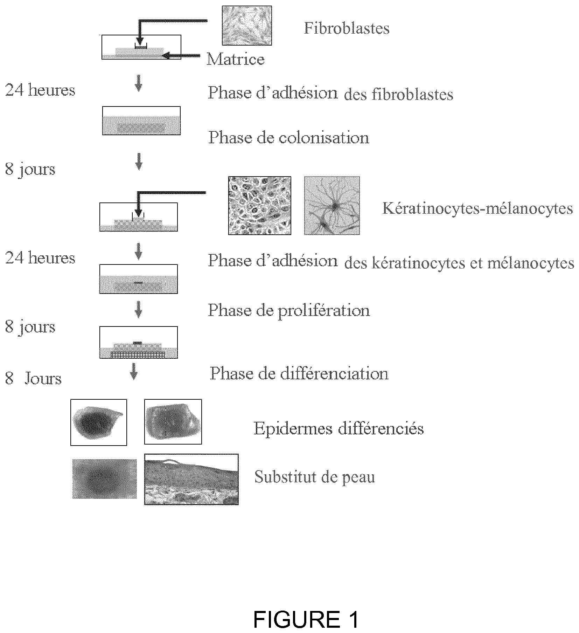

La figure 1 représente un schéma des étapes permettant l'obtention d'un substitut de peau.

Dans le présent exemple, deux substituts de peau obtenus présentaient des rapports (kératinocytes + mélanocytes)/fibroblastes de 13,3. Pour le rapport de 13,3, lors des étapes de dépôt des fibroblastes et du mélange kératinocytes/mélanocytes les quantités de cellules ensemencées étaient respectivement de 15000 pour les fibroblastes, de 10 000 pour les mélanocytes et de 190 000 pour les kératinocytes.

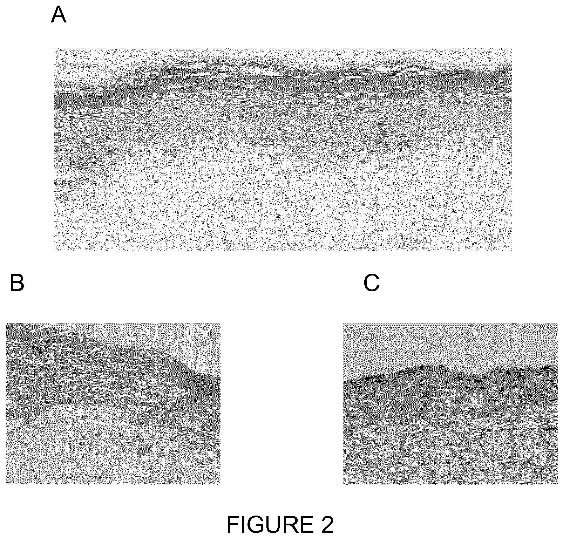

Une fois le substitut obtenu, il a été fixé dans du formol 4%, inclus en paraffine puis une coupe de 4 µm a été effectuée, puis une coloration hématoxyline-éosine a été effectuée afin de marquer les différentes couches de la peau. Une observation au microscope optique à un grossissement de 40x a été réalisée. Le microscope étant couplé à une caméra CCD (Nikon, logiciel NIS element Br) des photographies des observations ont été réalisées. La figure 2B représente une photographie en microscopie optique d'un substitut de peau obtenu selon le procédé dans lequel le ratio (kératinocytes + mélanocytes)/fibroblastes était de 13,3. La figure 2C représente une photographie en microscopie optique d'un substitut de peau obtenu selon le procédé dans lequel le ratio (kératinocytes + mélanocytes)/fibroblastes était de 6,7 et la figure 2A une photographie en microscopie optique d'une biopsie de peau normale. Les figures 6A et 6B représentent également des photographies en microscopie optique d'équivalent de peau obtenu selon le procédé dans lequel le ratio (kératinocytes + mélanocytes)/fibroblastes était de 13,3.

Par ailleurs, un marquage immunohistochimique du substitut obtenu selon le procédé dans lequel le ratio (kératinocytes + mélanocytes)/fibroblastes était de 13,3 et d'une peau in-vivo a été effectué selon le procédé décrit dans Salducci, M., André, N., Guéré, C., Martin, M., Fitoussi, R., Vié, K., and Cario-André, M. (2014). Factors secreted by irradiated aged fibroblasts induce solar lentigo in pigmented reconstructed epidermis. Pigment Cell Melanoma Res. 27, 502-504 [12] ou Simon, D., Daubos, A., Pain, C., Fitoussi, R., Vié, K., Taieb, A., de Benetti, L., and Cario-André, M. (2013). Exposure to acute electromagnetic radiation of mobile phone exposure range alters transiently skin homeostasis of a model of pigmented reconstructed epidermis. Int. J. Cosmet. Sci. 35, 27-34 [13] afin d'identifier dans le substitut la présence de mélanocytes, la production de la lame basale comprenant notamment du collagène IV (figure 6D (2)), la capacité de prolifération des cellules au niveau de la lame basale (figure 6D (1)) et la présence de mélanocytes (figure 6C). Les figures 6 E et 6F représentent des photographies en microscopie optique de peau in-vivo après le marquage immunohistochimique des mélanocytes en position basale (figure 6 E, zones claires), le marquage du collagène IV (figure 6F (2)) et de la p63 marqueur de prolifération (figure 6 F (1)). Il apparait clairement sur les figures 6C et 6D que le substitut de peau selon l'invention comprend des mélanocytes, une lame basale tel que démontré par la présence du collagène IV au niveau de laquelle les cellules présentes sont hautement prolifératives comme pour la peau in-vivo (figures 6 E et 6 F).

Tel que représenté sur ces photographies, le substitut obtenu par le procédé a une structure identique à celle de la peau in vivo.

### Exemple 2 : Greffe d'un substitut de derme selon l'invention sur souris

Dans cet exemple, le substitut de peau utilisé était le substitut obtenu comme décrit dans l'exemple 1 avec des cellules obtenues à partir de plasties mammaires avec le ratio (kératinocytes + mélanocytes)/fibroblastes de 13,3. Les souris utilisées étaient des souris nude swiww nu/nu provenant du Jackson Lab.

Dans cet exemple, des greffes ont été effectuées sur 10 souris en parallèle.

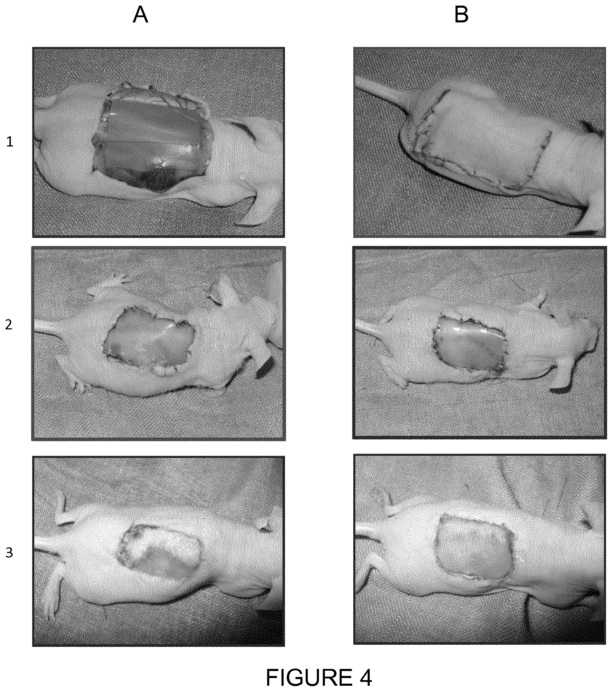

Une incision des souris a été effectuée à l'aide d'un scalpel sur une zone de 5 cm² afin de supprimer le derme et l'épiderme, un nettoyage de la zone scarifiée a été effectué avec du sérum physiologique. Un substitut de peau préalablement préparé et a été appliqué sur la zone scarifiée afin de la recouvrir (figure 4 A, ligne 1) puis la zone greffée a été fermée avec la peau de la souris qui a été suturée (figure 4 B, ligne 1).

Les souris ont été conservées dans une animalerie spécialisée seule par cage le temps que la greffe prenne puis à 5 dans des cages de taille adéquate.

Après 2 semaines, une observation de la prise de la greffe a été effectuée en retirant le capot de peau de la souris (figure 4 A, ligne 2). En d'autres termes, la peau suturée de la souris a été désuturée, éliminée et le substitut de derme greffé a été mis à jour pour observation visuelle (figure 4 B, ligne 2).

Après 3 semaines, une observation visuelle de la prise de la greffe a été effectuée (figures 4 B et B, ligne 3).

Tel que démontré sur la figure 4, il apparait clairement que le substitut de derme peut être avantageusement appliqué, notamment pour une greffe sans induction d'effets secondaires.

En outre, afin d'étudier l'état structurel du substitut de derme après trois semaines post application, un prélèvement a été effectué par découpe au scalpel du substitut. Le substitut a été fixé dans du formol 4%, inclus en paraffine puis une coupe de 4 µm a été effectuée, puis une coloration hématoxyline-éosine a été effectuée afin de marquer les différentes couches de la peau. Une observation au microscope optique à un grossissement de 40x a été réalisée. Le microscope étant couplé à une caméra CCD (Nikon, logiciel NIS element Br) des photographies des observations ont été réalisées.

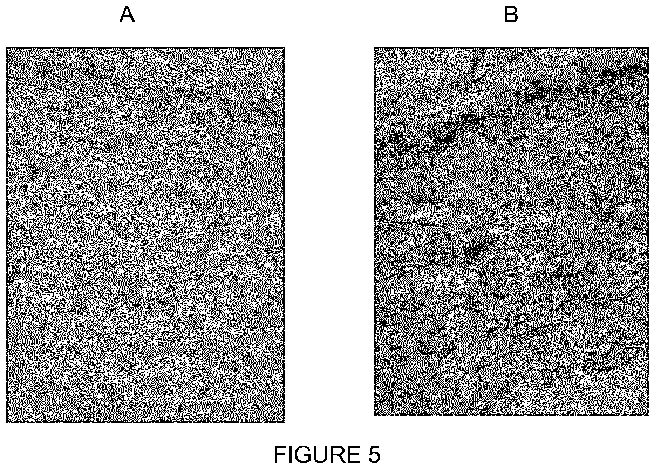

La figure 5 représente les photographies en microscopie optique de substituts greffés. En particulier, la figure 5 A, montre le substitut colonisé par les fibroblastes murins qui sont peu nombreux et qui n'ont pas commencé à remanier le collagène. La figure 5 B montre le substitut colonisé avec les fibroblastes humains avant greffe. On observe un nombre important de fibroblastes et un remaniement de la matrice notamment dans la zone superficielle avec des trousseaux plus épais de collagène. Le présent exemple démontre donc clairement que la présence de l'ensemble des cellules permet l'obtention d'un substitut facilement intégrable, qui ne se dégrade pas dans le temps et qui présente une maturation après greffe, qui est accéléré en présence de fibroblastes dans la matrice.

### Liste de références

**1.** Pendaries V et al, siRNA-mediated allele-specific inhibition of mutant type VII collagen in dominant dystrophic epidermolysis bullosa.JID 2012, Jun;132(6):1741-3.
**2.** Petek LM et al "Efficient KRT14 targeting and functional characterization of transplanted human keratinocytes for the treatment of epidermolysis bullosa simplex". Mol ther 2010, Sep; 18 (9):1624-32.
**3.** Kogut et al, "Differentiation of human induced pluripotent stem cells into a keratinocyte lineage" Methods Mol Biol 2014, 1195:1-12.
**4.** Ohta et al, "Generation of human melanocytes from induced pluripotent stem cells" Methods Mol Biol, 2013; 989:193-215.
**5.** Revilla et al, "Current advances in the generation of human iPS cells: implications in cell-based regenerative medicine." J Tissue Eng Regen Med, 2015, Mar 11.
**6.** Bell et al, 1979
**7.** Boyce ST et al, "Structure of a collagen-GAG dermal skin substitute optimized for cultured human epidermal keratinocytes", 1988 Oct; 22 (10):939-57.
**8.** Hafemann et al, "Use of a collagen/elastin-membrane for the tissue engineering of dermis." Burns 1999, Aug;25(5):373-84.
**9.** Wonhye Lee et al. "Multi-layered culture of human skin fibroblasts and keratinocytes through three-dimensional freeform fabrication." Biomaterials, 2009, Mar;30(8):1587-95
**10.** Peña, and al., J Oral and Maxillofacial Surgery, 70:10 10, 2012
**11.** E.Dantzer, F. Braye "Reconstructive surgery using an artificial dermis (Integra): results with 39 grafts." Br J Plast Surg, 54:8 8, 2001.
**12.**Salducci, M., André, N., Guéré, C., Martin, M., Fitoussi, R., Vié, K., and Cario-André, M. (2014). Factors secreted by irradiated aged fibroblasts induce solar lentigo in pigmented reconstructed epidermis. Pigment Cell Melanoma Res. 27, 502-504
**13.**Simon, D., Daubos, A., Pain, C., Fitoussi, R., Vié, K., Taieb, A., de Benetti, L., and Cario-André, M. (2013). Exposure to acute electromagnetic radiation of mobile phone exposure range alters transiently skin homeostasis of a model of pigmented reconstructed epidermis. Int. J. Cosmet. Sci. 35, 27-34

## Revendications

1. Procédé de préparation d'un substitut de peau comprenant les étapes suivantes :
**a.** culture de fibroblastes dans un milieu M1 de culture de fibroblastes ;
**b.** ensemencement d'une matrice comprenant du collagène avec des fibroblastes issus de l'étape a ;
**c.** culture des fibroblastes ensemencés dans la matrice comprenant du collagène dans un milieu M2 de culture de fibroblastes comprenant de l'acide ascorbique ou un ascorbate ou un dérivé de ceux-ci, la matrice et les fibroblastes cultivés formant un substitut de derme ;
**d.** culture de mélanocytes dans un milieu M3 de culture de mélanocytes ;
**e.** culture de kératinocytes dans un milieu M4 de culture de kératinocytes ;
**f.** mélange de mélanocytes obtenus à l'étape d avec des kératinocytes obtenus à l'étape e ;
**g.** ensemencement du substitut de derme obtenu à l'étape c par le mélange obtenu à l'étape f ; dans lequel l'ensemencement à l'étape g est réalisé avec un ratio (kératinocytes + mélanocytes)/fibroblastes de 9 à 19, et
**h.** culture du substitut de derme ensemencé à l'étape g dans un milieu M5 de culture de peau formant ainsi le substitut de peau.

2. Procédé selon la revendication 1, dans lequel le milieu M5 comprend de l'acide hyaluronique ou un hyaluronate ou un dérivé de ceux-ci.

3. Procédé selon la revendication 2, dans lequel le milieu M5 comprend de l'acide ascorbique ou un ascorbate ou un dérivé de ceux-ci.

4. Procédé selon l'une quelconque des revendications 1 à 3, dans lequel le mélange de mélanocytes et de kératinocytes de l'étape f est réalisé avec un ratio mélanocytes / kératinocytes de 1/20 à 1/15.

5. Procédé selon l'une quelconque des revendications précédentes, dans lequel l'ensemencement à l'étape b est réalisé à une densité de 20 000 à 50 000 fibroblastes / cm² de surface de la matrice comprenant du collagène.

6. Procédé selon l'une quelconque des revendications précédentes, dans lequel l'étape c. comprend une première étape c' de culture de 18 à 28 heures en présence d'un milieu M2¹ de culture de fibroblastes ne comprenant ni acide ascorbique ni ascorbate, une deuxième étape c" de culture d'au moins 2 jours en présence d'un milieu M2² de culture de fibroblastes comprenant de l'acide ascorbique ou un ascorbate ou un dérivé de ceux-ci.

7. Procédé selon l'une quelconque des revendications précédentes, dans lequel l'étape h. comprend :
- une première étape h.' de culture de 6 à 24 heures en présence d'un milieu M5¹ de culture ne comprenant ni acide hyaluronique, ni hyaluronate, ni acide ascorbique et ni ascorbate,
- une deuxième étape h." de culture d'au moins 2 jours présence d'un milieu M5² de culture comprenant de l'acide hyaluronique ou un hyaluronate ou un dérivé de ceux-ci, et
- une troisième étape h.'" de culture d'au moins 2 jours dans un milieu M5³ comprenant de l'acide hyaluronique ou un hyaluronate ou un dérivé de ceux-ci, et de l'acide ascorbique ou un ascorbate ou un dérivé de ceux-ci.

8. Substitut de peau susceptible d'être obtenu par la mise en œuvre du procédé défini dans l'une quelconque des revendications précédentes.

9. Substitut de peau selon la revendication 8, dans lequel les fibroblastes sont autologues d'un individu en vue d'une greffe dudit substitut de peau sur ledit individu.

10. Substitut de peau selon la revendication 9, dans lequel les fibroblastes, les mélanocytes et kératinocytes sont autologues d'un individu.

11. Substitut de derme susceptible d'être obtenu à l'étape c. du procédé défini dans l'une quelconque des revendications précédentes.

12. Greffon constitué d'un substitut de peau selon la revendication 9 ou 10 ou substitut de derme selon la revendication 11.

13. Greffon selon la revendication 12 pour une utilisation comme moyen de traitement d'un désordre cutané et/ou d'une perte de substance cutanée.

14. Greffon pour une utilisation selon la revendication 13 dans lequel le désordre cutané et/ou une perte de substance cutanée est choisi dans le groupe comprenant une brûlure, un défaut de cicatrisation, une plaie chronique, un désordre pigmentaire, un hémangiome et un cancer cutané.

15. Kit pour la mise en œuvre du procédé selon l'une quelconque des revendications 1 à 7 comprenant un milieu M1 de culture de fibroblaste, une matrice comprenant du collagène, un milieu M2 de culture des fibroblastes comprenant de l'acide ascorbique ou un ascorbate ou un dérivé de ceux-ci, un milieu M3 de culture de mélanocytes, un milieu M4 de culture de kératinocytes et un milieu M5 de culture de peau.

16. Kit selon la revendication 15 dans lequel les milieux M1 à M5 sont indépendamment des milieux de grade clinique.

## Patentansprüche

1. Verfahren zur Herstellung eines Hautersatzes, umfassend die folgenden Schritte:
a) Kultivieren von Fibroblasten in einem Fibroblastennährmedium M1;
b) Beimpfen einer kollagenhaltigen Matrix mit aus dem Schritt a) stammenden Fibroblasten;
c) Kultivieren der in die kollagenhaltige Matrix geimpften Fibroblasten in einem Fibroblastennährmedium M2, wobei das Medium M2 Ascorbinsäure oder ein Ascorbat oder ein Derivat davon enthält, and wobei die Matrix und die kultivierten Fibroblasten einen Dermisersatz bilden;
d) Kultivieren von Melanozyten in einem Melanozytennährmedium M3;
e) Kultivieren von Keratinozyten in einem Keratinozytennährmedium M4;
f) Mischen der im Schritt d) erhaltenen Melanozyten mit den im Schritt e) erhaltenen Keratinozyten;
g) Beimpfen des im Schritt c) erhaltenen Dermisersatzes mit dem in Schritt f) erhaltenen Gemisch, wobei das Beimpfen in Schritt g) mit einem Verhältnis (Keratinozyten + Melanozyten) / Fibroblasten von 9 bis 19 durchgeführt ist; und
h) Kultivieren des im Schritt g) beimpften Dermisersatzes in einem Hautnährmedium M5, wodurch der Hautersatz gebildet wird.

2. Verfahren nach Anspruch 1, wobei das Medium M5 Hyaluronsäure oder ein Hyaluronat oder ein Derivat davon umfasst.

3. Verfahren nach Anspruch 2, wobei das Medium M5 Ascorbinsäure oder ein Ascorbat enthält oder ein Derivat davon umfasst.

4. Verfahren nach einem der Ansprüche 1 bis 3, wobei das Gemisch von Melanozyten und Keratinozyten des Schritts f) mit einem Melanozyten/Keratinozyten-Verhältnis von 1/20 bis 1/15 erfolgt.

5. Verfahren nach einem der vorhergehenden Ansprüche, wobei das Beimpfen im Schritt b mit einer Dichte von 20 000 bis 50 000 Fibroblasten/cm² Oberfläche der kollagenhaltigen Matrix erfolgt.

6. Verfahren nach einem der vorhergehenden Ansprüche, wobei der Schritt c einen ersten Kultivierungsschritt c' von 18 bis 28 Stunden in Gegenwart eines Fibroblastennährmediums M2¹, das weder Ascorbinsäure noch Ascorbat enthält, und einen zweiten Kultivierungsschritt c" von mindestens 2 Tagen in Gegenwart eines Fibroblastennährmediums M2², das Ascorbinsäure oder ein Ascorbat enthält, umfasst.

7. Verfahren nach einem der vorhergehenden Ansprüche, wobei der Schritt h) Folgendes umfasst:
- einen ersten Kultivierungsschritt h' von 6 bis 24 Stunden in Gegenwart eines Nährmediums M5¹, das weder Hyaluronsäure, noch Hyaluronat, noch Ascorbinsäure und auch kein Ascorbat enthält,
- einen zweiten Kultivierungsschritt h" von mindestens 2 Tagen in Gegenwart eines Nährmediums M5², das Hyaluronsäure oder ein Hyaluronat enthält, und
- einen dritten Kultivierungsschritt h'" von mindestens 2 Tagen in einem Nährmedium M5³, das Hyaluronsäure oder ein Hyaluronat oder ein Derivat davon und Ascorbinsäure oder ein Ascorbat oder ein Derivat davon umfast.

8. Hautersatz, der durch Durchführen des in einem der vorhergehenden Ansprüche definierten Verfahrens erhalten wird.

9. Hautersatz nach Anspruch 8, wobei die Fibroblasten autolog sind, von einem Individuum, dem der Hautersatz transplantiert werden soll.

10. Hautersatz nach Anspruch 9, wobei die Fibroblasten, die Melanozyten und die Keratinozyten autolog sind, von einem Individuum.

11. Dermisersatz, der durch Durchführen der Schritte c) in einem der vorhergehenden Ansprüche definierten Verfahrens erhalten wird.

12. Transplantat, bestehend aus einem Hautersatz nach Anspruch 9 oder 10, oder Dermisersatz nach Anspruch 11.

13. Transplantat nach Anspruch 12 für eine Verwendung als Mittel zur Behandlung einer Hauterkrankung und/oder eines Hautsubstanzverlusts.

14. Transplantat für eine Verwendung nach Anspruch 13, wobei die Hauterkrankung und/oder der Hautsubstanzverlust aus der Gruppe gewählt ist, die Verbrennung, Wundheilungsstörung, chronische Wunde, Pigmentstörung, Hämangiom und Hautkrebs umfasst.

15. Kit für die Durchführung des Verfahrens nach einem der Ansprüche 1 bis 7, umfassend ein Fibroblastennährmedium M1, eine kollagenhaltige Matrix, ein Fibroblastennährmedium M2 der Ascorbinsäure oder ein Ascorbat enthält, ein Melanozytennährmedium M3, ein Keratinozytennährmedium M4 und ein Hautnährmedium M5.

16. Kit nach Anspruch 15, wobei die Medien M1 bis M5 voneinander unabhängig Medien klinischer Güte sind.

## Claims

1. Method for preparation of a skin substitute comprising the following steps:
a. fibroblasts culture in a fibroblast culture medium M1;
b. seeding of a matrix comprising collagen with fibroblasts obtained from step a;
c. culture of seeded fibroblasts in a matrix comprising collagen in a fibroblasts culture medium M2 comprising ascorbic acid or an ascorbate or a derivative thereof, the matrix and the cultivated fibroblasts forming a dermis substitute;
d. melanocytes culture in a melanocytes culture medium M3;
e. keratinocytes culture in a keratinocytes culture medium M4;
f. mix of melanocytes obtained in step d with keratinocytes obtained in step e;
g. seeding of the dermis substitute obtained in step c with the mix obtained in step f; wherein seeding in step g is carried out with a (keratinocytes + melanocytes)/fibroblasts ratio from 9 and 19, and
h. dermis substitute culture seeded in step g in a skin culture medium M5 thus forming the skin substitute.

2. Method according to claim 1, wherein the medium M5 comprises hyaluronic acid or a hyaluronate or a derivative thereof.

3. Method according to claim 2, wherein the medium M5 comprises ascorbic acid or an ascorbate or a derivative thereof.

4. Method according to any one of claims 1 to 3, wherein the mix of melanocytes and keratinocytes in step f is made with a melanocytes / keratinocytes ratio from 1/20 and 1/15.

5. Method according to any one of the previous claims, wherein seeding done in step b is done at a density of from 20 000 to 50 000 fibroblasts / cm² of surface area of the matrix comprising collagen.

6. Method according to any one of the previous claims, in which step c. comprises a first culture step c' for 18 to 28 hours in the presence of a fibroblasts culture medium M2¹ comprising neither ascorbic acid nor ascorbate, a second culture step c" of at least 2 days in the presence of a fibroblasts culture medium M2² comprising ascorbic acid or an ascorbate or derivative thereof.

7. Method according to any one of the previous claims, wherein step h. comprises:
- a first 6 to 24 hour culture step h.' in the presence of a culture medium M5¹ comprising neither hyaluronic acid, nor hyaluronate, nor ascorbic acid nor ascorbate,
- a second culture step h." of at least 2 days in the presence of a culture medium M5² comprising hyaluronic acid or a hyaluronate, and
- a third culture step h."' of at least 2 days in a medium M5³ comprising hyaluronic acid or a hyaluronate or a derivative thereof, and ascorbic acid or an ascorbate or a derivative thereof.

8. Skin substitute obtainable by implementation of the method defined according to any one of the previous claims.

9. Skin substitute according to claim 8, wherein the fibroblasts are autologous of an individual in preparation for a graft of said skin substitute on said individual.

10. Skin substitute according to claim 9, wherein the fibroblasts, melanocytes and keratinocytes are autologous of an individual.

11. Dermis substitute obtainable by implementation at step c. of the method defined according to any one of the previous claims.

12. Graft constituted of a skin substitute according to claim 9 or 10 or a dermis substitute according to claim 11.

13. Graft according to claim 12 for use as a treatment means of a skin disorder and/or a loss of skin substance.

14. Graft for use according to claim 13 wherein the skin disorder and/or loss of skin substance is selected from the group comprising a burn, a healing defect, a chronic wound, a pigmentary disorder, a hemangioma and a skin cancer.

15. Kit for use of the method according to any one of claims 1 to 7 comprising a fibroblast culture medium M1, a matrix comprising collagen, a fibroblast culture medium M2 in the matrix comprising ascorbic acid or an ascorbate or a derivative thereof, a melanocytes culture medium M3, a keratinocytes culture medium M4 and a skin culture medium M5.

16. Kit according to claim 15 wherein the media M1 to M5 are independently clinical grade media.
